# EUROPEAN PATENT SPECIFICATION

(11) **EP 3 076 946 B1**
(45) Date of publication and mention of the grant of the patent: **07.01.2026**
(21) Application number: 14828594.3
(22) Date of filing: 08.12.2014
(51) Int. Cl.: A61K 9/00

(54) **COMPOSITIONS COMPRISING ANTIOXIDANT, FLUID DISPENSERS, AND METHODS INVOLVING THE SAME**
ZUSAMMENSETZUNGEN MIT ANTIOXIDATIONSMITTEL, FLÜSSIGKEITSSPENDER UND VERFAHREN DAMIT
COMPOSITIONS COMPRENANT UN ANTIOXYDANT, DES DISTRIBUTEURS DE LIQUIDE, ET PROCÉDÉS IMPLIQUANT CEUX-CI

(30) Priority: 06.12.2013 US 201361913139 P
(43) Date of publication of application: 12.10.2016
(73) Proprietor: Alzet, LLC, N Lafayette, Indiana 47904 (US)
(72) Inventor: SONG, Xiaoping, Saratoga, CA 95070 (US); PEER, Clarissa, San Jose, CA 95124 (US); YUM, Su Ii, Los Altos, CA 94024 (US)
(74) Representative: J A Kemp LLP
(86) International application number: PCT/US2014/069135
(87) International publication number: WO 2015/085312

(56) References cited:
- WO-A1-91/15196
- US-A- 4 627 850
- US-A- 4 940 465
- US-A1- 2006 159 763
- US-A1- 2007 003 624

## Description

### BACKGROUND TO THE INVENTION

US 4,627,850 is concerned with an osmotic capsule for delivering a beneficial agent formulation to an environment of use. The osmotic capsule comprises an outer semipermeable wall surrounding and laminating an inner capsule wall formed of a different polymeric composition than the outer wall. The walls define an interior space containing the beneficial agent formulation. A passageway through the walls connects the exterior of the osmotic capsule with the interior of the osmotic capsule.

US 4,940,465 is concerned with a dispenser for delivering a beneficial agent to an environment of use. The dispenser comprises a wall that surrounds an internal space comprising a first means in the dispenser for changing from a dispenser state to an environment of use state on leaving the dispenser, a beneficial agent in the first means, and a second means in the dispenser for aiding in displacing the first means from the dispenser.

WO 91/15196 is concerned with an osmotic system for delivering a beneficial agent formulation to an environment of use. The osmotic system comprises an outside semipermeable wall, a middle osmotically active layer, a capsule comprising a liquid formulation comprising a beneficial agent, and a passageway for dispensing the beneficial agent from the osmotic system.

### SUMMARY OF THE INVENTION

The present invention provides fluid dispensers comprising antioxidant according to claim 1. Other features and advantages of the present invention will be set forth in the description of invention that follows, and in part will be apparent from the description or may be learned by practice of the invention. The invention will be realized and attained by the compositions and methods particularly pointed out in the written description and claims hereof.

The present invention is directed to a dispenser comprising: a flexible container having a closed end and an opposed open end; an osmotically-effective solute composition encapsulating at least a portion of the container, the solute composition comprising (a) antioxidant in an amount ranging from 0.5 wt% to 20 wt%, based on weight of the solute composition, wherein the antioxidant comprises at least one member selected from butylated hydroxytoluene (BHT), butylated hydroxyanisole (BHA), thioethers, histidine, cysteine, tryptophan, tyrosine, ascorbic acid, vitamin E, ascorbyl palmitate, erythorbic acid, potassium metabisulfite, propyl gallate, sodium ascorbate, sodium bisulfate, sodium metabisulfite, sodium sulfite, sodium formaldehyde sulfoxylate, and thymol, (b) a polymer comprising at least one of polyethylene oxide and polyethylene glycol, and (c) a salt; a semipermeable membrane encapsulating the osmotically-effective solute composition; a cap having a sealing surface adapted for sealing engagement with the open end of the container; and a port extending from an interior of the container and through the cap.

A method of administering an active agent to a subject (this method not being recited in the claims) comprises implanting an osmotic pump in the subject, the osmotic pump comprising a solute composition comprising antioxidant in an amount of at least 0.5 wt%, based on weight of the solute composition; and releasing the active agent from the implanted pump into the subject.

A method of making an osmotic pump comprising a solute composition comprising antioxidant in an amount of at least 0.5 wt%, based on weight of the solute composition, (this method not being recited in the claims) comprises irradiating the osmotic pump with γ-irradiation.

### BRIEF DESCRIPTION OF THE DRAWINGS

The present invention is further described in the description of invention that follows, in reference to the noted plurality of non-limiting drawings, wherein:
FIG. 1 is an elevational, exploded, sectional view of one embodiment of an osmotic pump dispenser of the invention;
FIG. 2 is an enlarged sectional view of the pump dispenser of FIG. 1;
FIG. 3 is a sectional view of the inner bag of the dispenser of FIG. 1;
FIG. 4 is a sectional view of an alternative inner bag;
FIG. 5 is an enlarged, top plan view of the flow moderator of the dispenser of FIG. 1;
FIG. 6 is an elevational, exploded, partly sectional view of another embodiment of the dispenser of the invention; and
FIG. 7 is a sectional view of the dispenser of FIG. 6.

### DESCRIPTION OF THE INVENTION

Unless otherwise stated, a reference to a compound or component includes the compound or component by itself, as well as in combination with other compounds or components, such as mixtures of compounds.

As used herein, the singular forms "a," "an," and "the" include the plural reference unless the context clearly dictates otherwise.

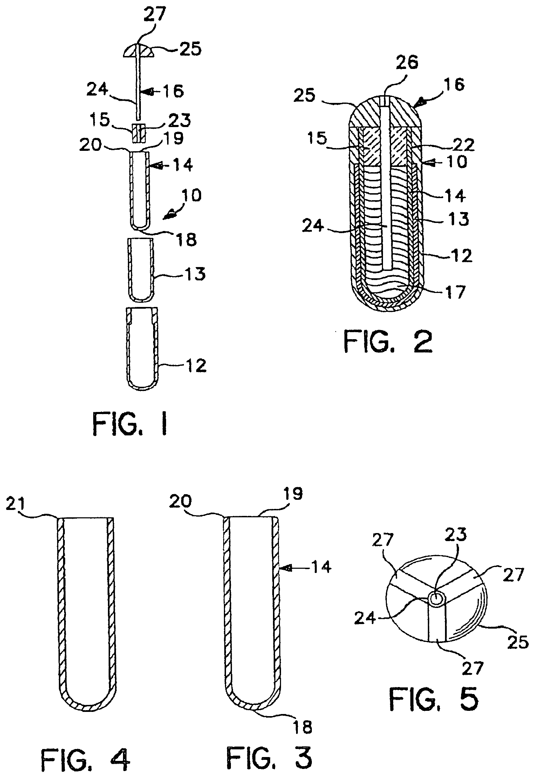

FIGS. 1 and 2 illustrate an osmotically driven fluid dispenser, generally designated 10. The basic components of dispenser 10 are an outer, shape-retaining, semipermeable membrane 12, an intermediate layer 13 of an osmotically effective solute, an inner, flexible container 14, a plug 15, and a flow moderator, generally designated 16.

Container 14 may be formed as a bag adapted to contain a fluid composition, such as an active agent composition 17, as best seen in FIG. 2, in fluid form. The term "active agent" as used herein means any compound or mixture of compounds that can be dispensed to produce a predetermined beneficial and useful result. Exemplary active agents are recited in U.S. Pat. No. 4,320,758 at Col. 2, lines 24-37. The term "bag" means any suitable container having a closed end and an open end, as described more fully below.

Container 14 is typically substantially impermeable to the active agent composition and typically compatible with the active agent composition. By "compatible", it is meant that the bag should not be corroded, solubilized, or otherwise affected deleteriously by the composition. Additionally, when the composition is a drug or the like, the composition should not be significantly contaminated by the bag, such as by the extraction of leachables from the material forming the bag. The container is usually capable of maintaining the concentrations of both inactive and active agents.

Container 14 may be made from elastomeric compositions that may be formed into thin sheets. The elastomeric properties of the bag composition and the thickness of the bag wall should be such as to cause the bag to readily collapse inwardly when a force is applied to the bag exterior. Such elastomeric compositions are disclosed, e.g., in U.S. Pat. No. 3,760,984 at Col. 5, line 40 to Col. 7, line 37, and in commonly owned Canadian Pat. No. 949,513 at p. 10, line 28 to p. 11, line 7.

For instance, container 14 may be a heat shrinkable, polymeric material that collapses on the application of force thereto. The polymeric material may be selected from the class of heat shrinkable polymeric films in the form of tubes, spheres, ellipsoids, envelopes, films, laminates, and other geometric shapes and fabricated structures is in one embodiment a material that has been prepared by inducing strong molecular orientation by uni-axially or bi-axially stretching of the film, which operation, preferably, can be preceded by the introduction of intermolecular primary valence cross-linkage by chemical or radiation processes. The degree of cross-linking, when employed, should be sufficient to impart to the film a thermoset character, which can be conveniently defined as the ability to exhibit a minimum tensile strength of about 3.4 bar (50 lbs./in.²) at a temperature of 300°F. By "heat shrinkable" is meant in this embodiment that the film can contract by at least 10 percent and typically from about 25 percent to 75 percent of its stretched dimension in one or more directions upon heating. The material may be expanded or stretched mechanically, hydraulically, or pneumatically, either uni-axially or bi-axially, at room temperature or elevated temperatures, and then may be set or fixed, or "frozen", into this expanded, high energy state. Procedures for accomplishing this are known in the polymer fabrication art. For example, in the manufacture of bi-axially oriented, heat shrinkable flim, the film may be prepared by extrusion through a shaping die with a long, narrow horizontal slit of such width as to give the desired film thickness. As the hot ribbon of polymeric material issues from the die, it may be gripped along its two edges by tenter hooks which tend to stretch the film along its width and to stretch it in a forward direction at the same time. This operation imparts bi-axial orientation and yields a film with equal shrinkage along both axes. Typically, such a film will have a potential shrinkage of 50 percent in both directions. Not only is the rate of stretching important in achieving this result, but also the rate of cooling and the temperature profile during the stretching are important.

In the manufacture of one type of heat shrinkable tubing for use in the present invention, the polymer may first be prepared in tubular shape, preferably by extrusion through a die of the desired cross-sectional configuration. The tubing can then be subjected to ionizing radiation consisting of a stream of high energy electrons as delivered by a van de Graaff generator or other electron accelerating equipment. Or the tubing can be treated with gamma rays as emanating from cobalt-60. The dosage delivered can vary, depending upon the polymer system, e.g., from 5,000 to 1,000,000 Gy (0.5 to 100 megarads) to achieve the desired degree of intermolecular cross-linkage. The tubing may then be subjected to uni-axial molecular orientation by drawing it, optimally in a warm or heated condition, over an appropriately shaped mandrel, which increases the cross-sectional area by a factor of 2 to 16. The polymer may be selected from classes which tend to have high intermolecular attraction and tend to remain in the high energy, stretched state until heated above a temperature at which these intermolecular attractions are melted or released. The "memory" or tendency to recover back to the unstretched state is encouraged by the cross-linkage which was introduced by the earlier radiation treatment.

Polymeric membranes used to form the container 14 are preferably cross-linked prior to stretching. The chemical cross-linking of these polymers can be achieved by incorporation of various cross-linking agents such as peroxides, sulfur, metallic oxides, selenium, tellerium, diamines, diisocyanates, alkyl phenol disulfides, p-quinone dioxime, tetra-chloro-p-benzoquinone, tetra alkyl thiuram disulfides, 4,4'-dithiomorpholine, sulfur dichloride, and the like, into the polymer followed by a period of heating. Alternatively, cross-linking or vulcanization can be achieved by use of high energy electron-beam radiation such as is provided by a van de Graaff generator or other types of electron accelerators, or by gamma ray emitters, or by X-ray generators.

In another embodiment pre-oriented shrinkable materials suitable for forming the container 14 comprise oriented film of vinyl chloride polymer which has a Young's modulus of elasticity in both directions of at least 14,000 bar (200,000 p.s.i.; 14,000 kg/cm²) at 23°C, a shrinkage of at most 35 percent at 10.3 bar (150 p.s.i.; 10.5 kg/cm²) at any temperature. The films preferably have shrink tensions not exceeding 6.9 bar (100 p.s.i.; 7 kg/cm²) at any temperature. The most preferred film is a rigid (i.e. unplasticized) polyvinyl chloride film which is 0.01 to 0.95 mm thick and has been bi-axially oriented so that it has a shrinkage in both directions of at most about 20 percent, especially 15 to 20 percent, e.g. about 20 percent. Films having low degrees of orientation or shrink in one direction only, such as are produced directly by some extrusion methods, can be used in accordance with the invention, but typically involve the use of rather high film temperatures, near the melting point of the polymer, in order sufficiently to shrink the film. Accordingly it is preferred to use films which have been bi-axially oriented so that they have percent shrinkages at 100°C in both directions of 5 to 35 percent, especially 15 to 25 percent, particularly 15 to 20 percent, and have shrink tensions not exceeding 10.3 bar (150 p.s.i.; 10.5 kg/cm²) and preferably not exceeding 6.9 bar (100 p.s.i.; 7.0 kg/cm²) at any temperature. They can be satisfactorily used in shrink packaging procedures in which the film only has to reach a maximum temperature of 120°C and for regular objects 100°C or even less.

The vinyl chloride polymer shrinkable materials used herein include homopolymers and copolymers such as vinyl chloride and vinyl acetate, styrene, acrylonitrile, dialkyl fumarate or maleate, or alkyl acrylate or methacrylate, vinyl acetate and vinylidene chloride, blends of polyvinyl chloride with chlorinated polyethylene or terpolymer, and the like. Other heat shrinkable materials include vinylidene chloride, copolymers of vinylidene chloride of 20 to 80 percent vinylidene chloride, copolymers of vinylidene chloride and vinyl chloride, and the like.

In some embodiments, the container 14 is comprised of at least one polymer selected from polyethylene (e.g., low density polyethylene or ultra low density polyethylene), nitrile rubber, silicone rubber, and ethylene vinyl acetate (EVA) copolymers.

In some embodiments, Kraton^{™} polymer is used to form the container 14. Kraton^{™} polymers are styrenic block copolymers consisting of polystyrene blocks and rubber blocks. The rubber blocks consist of polybutadiene, polyisoprene, or their hydrogenated equivalents.

Container, e.g., bag, 14 may be elongated and generally cylindrical and closed at its end 18 and open at its opposite end 19. Edge 20 at open end 19 of the bag may be arcuate, as seen best in the exploded view of the inner bag in FIG. 3. The curvature of edge 20 can be selectively varied. Alternatively, the edge may form a sharp point, as does the edge 21 with a 90° angle seen in FIG. 4. The arcuate edge may reduce the development of cracks or fissures in the outer membrane 12. Accordingly, in one embodiment of the invention, edge 20 of the inner bag is at an angle of other than 90°, and in a preferred embodiment has an angle of less than 90°. In terms of radius of curvature, in a preferred embodiment of the invention, edge 20 has a radius of curvature of greater than 0.01 inches (0.254 mm), such as a radius of curvature of greater than 0.02 inches (0.508 mm). The upper boundary of the radius of curvature of the edge is less critical, since curvature may minimize the development of fissures. Devices may be manufactured where the radius of curvature of the edge of concern is in the range of 2.03 mm (0.08 inches) to 2.29 mm (0.09 inches). Thus, in one embodiment a device having an edge with a radius of curvature ranging from 0.254-2.29 mm (0.01 inch to 0.09 inch), such as ranging from 0.254-2.03 mm (0.01 inch to 0.08 inch) or ranging from 0.254-1.78 mm (0.01 inch to 0.07 inch), is contemplated.

Returning now to FIGS. 1 and 2, container, e.g., bag, 14 is partly encapsulated by layer 13 of an osmotically effective solute composition such that a band 22 of the exterior of bag 14 proximate to open end 19 is not covered by layer 13. The purpose of layer 13 is to imbibe water across membrane 12 into the space between the exterior of bag 14 and the inner surface of membrane 12, that is, the space occupied by layer 13. Osmotically effective solute compositions that may be used to form layer 13 are disclosed in U.S. Pat. No. 3,760,984 at Col. 7, line 38 to Col. 8, line 2 and in Canadian Pat. No. 949,513 at p. 18, lines 22-27 and in U.S. Pat. No. 4,320,758 at Col. 3, line 2 to line 38.

For instance, various osmotically effective solutes including organic and inorganic compounds are advantageously used for layer 13 to act as a means for generating osmotic pressure. Suitable solutes exhibit an osmotic pressure gradient against an external fluid across the semi-permeable membrane which membrane is substantially impermeable to the passage of the osmotically effective solute to prevent loss thereof through the membrane. Various osmotically effective solutes include compounds such as magnesium sulfate, magnesium chloride, sodium chloride, lithium chloride, potassium sulfate, sodium carbonate, sodium sulfite, lithium sulfate, calcium bicarbonate, sodium sulfate, calcium sulfate, potassium acid phosphate, calcium lactate, magnesium succinate, tartaric acid, soluble carbohydrates such as raffinose, glucose, mixtures thereof, and the like.

Additionally, the solute can be used in a mixed form by mixing the compound with a binder. The solute in powdered, granular, piece and the like form, is homogenously or heterogenously dispersed in the binder which binder is soluble or insoluble but will release the solute on contact with wall material. Typical binders include polyethylene glycol, gelatin, agar, carboxycellulose, ethylmethylcellulose, polyvinyl alcohol, polyvinylpyrrolidone, soluble starch derivatives, and the like. Typical binders that can comprise about 1 to 50 percent of the composition include cellulose acetate, polyurethane, epoxides, and other binders that permit the free movement of fluid into the solute of the layered structure to permit the solute to increase in volume and generate osmotic pressure.

In the dispenser of the present invention the layer 13 comprises antioxidant. Antioxidant may be included to prevent aldehyde formation. For example, in some cases, the container may be sterilized with radiation, e.g., gamma radiation. Without the presence of antioxidant, the gamma radiation may cause materials in the layer 13 to form aldehydes. For instance, gamma irradiation may cause polyethylene glycol (PEG) and/or polyethylene oxide (PEO) to form aldehydes. It has been reported that radiation induced oxidation of solid polyethylene oxide generates several products. One of the main products is formaldehyde (Journal of Polymer Science: Polymer Chemistry Edition. V15, Issue 4 Page 781-798, April 1977). The aldehydes may then permeate through the bag 14. The aldehydes may then react with active and/or inactive agents in composition 17.

To minimize the effect of aldehydes, layer 13 in the dispenser of the present invention includes antioxidant. For instance, it may be desirable to include an antioxidant in order to provide radiation-stability.

Antioxidant is present in the solute composition in the dispenser of the present invention in an amount ranging from 0.5 wt% to 20 wt% based on weight of the solute composition. Thus, antioxidant may be added at, e.g., a concentration of at least about 1 wt%, or at least about 1.5 wt%, based on weight of layer 13. For instance, the amount of antioxidant may range from about 1 wt% to about 20 wt%, from about 5 wt% to about 20 wt%, or from about 10 wt% to about 20 wt%.

The antioxidants include, but are not limited to, butylated hydroxytoluene (BHT), butylated hydroxyanisole (BHA), thioethers, histidine, cysteine, tryptophan, tyrosine, ascorbic acid, vitamin E, ascorbyl palmitate, erythorbic acid, potassium metabisulfite, propyl gallate, sodium ascorbate, sodium bisulfate, sodium metabisulfite, sodium sulfite, sodium formaldehyde sulfoxylate, thymol, and mixtures thereof. Thioether antioxidants include, but are not limited to, those disclosed in U.S. Patent No. 6,989,138. Thioethers include, but are not limited to, methionine. In the dispenser of the present invention, the solute composition comprises antioxidant that comprises at least one member selected from butylated hydroxytoluene (BHT), butylated hydroxyanisole (BHA), thioethers, histidine, cysteine, tryptophan, tyrosine, ascorbic acid, vitamin E, ascorbyl palmitate, erythorbic acid, potassium metabisulfite, propyl gallate, sodium ascorbate, sodium bisulfate, sodium metabisulfite, sodium sulfite, sodium formaldehyde sulfoxylate, and thymol. The preferred antioxidant is BHT.

In the dispenser of the present invention, the solute composition also comprises both: (a) a polymer comprising at least one of polyethylene oxide and polyethylene glycol; and (b) a salt. In some embodiments, the solute layer 13 may comprise a salt (e.g., NaCl) in an amount ranging from 20 wt% to 60 wt% (e.g., 30 wt% to 55 wt% or 40 wt% to 50 wt%), polyethylene oxide in an amount ranging from 20 wt% to 55 wt% (e.g., 30 wt% to 50 wt% or 35 wt% to 45 wt%), polyethylene glycol in an amount ranging from 5 wt% to 25 wt% (e.g., 10 wt% to 20 wt%), and antioxidant (e.g., BHT) in an amount ranging from 0.1 wt% to 20 wt% (e.g., 0.5 wt% to 10 wt% or 1 wt% to 5 wt%).

Solute layer 13 is in turn be encapsulated by outer membrane 12. Membrane 12 also covers band 22 and forms a fluid tight seal therewith. At least a part of membrane 12 is permeable to water. Preferably all of membrane 12 is semipermeable. For instance, membrane 12 may be selectively permeable to water from outside of the device in FIG. 2. Membrane 12 is typically shape-retaining; that is, it is sufficiently rigid to be substantially undeformed by the hydrostatic pressure that is generated in the space between its inner surface and the exterior of bag 14 by the water imbibed by layer 13. The thickness and composition of membrane 12 affects the rate at which water will be imbibed through it by solute layer 13. Such membranes and compositions that may be used to form them are disclosed in U.S. Pat. No. 3,760,984 at col. 4, line 53 to col. 5, line 39 and in Canadian Pat. No. 949,513 at p. 9, line 24 to p. 10, line 27. However, preferred membranes are those formed from cellulose acetate butyrate, cellulose acetate propionate, and polymethylacrylate, either alone or in mixtures with each other, and optionally in mixture with ethyl cellulose, that are deposited out of solvent systems that are described in greater detail hereinafter. The outer membrane thickness typically will be between 0.0254 millimeters-1.27 millimeters (0.001-0.050 inches), and preferably 0.102 millimeters-0.762 millimeters (0.004-0.030 inches).

It will be appreciated that outer membrane 12 can include, if desired, a plasticizer to lend flexibility to the membrane. Plasticizers are well known to those of skill in the art and include, for example, triethyl citrate, tributyl citrate and poloxamers (brand name PLURONIC, product of BASF Corp., Mt. Olive, N.J.). Other materials for imparting flexibility and elongation properties to the membrane, for making the membrane less-to-nonbrittle and to render tear strength, include phthalate plasticizers such as dibenzyl phthalate, dihexyl phthalate, butyl octyl phthalate, straight chain phthalates of six to eleven carbons, di-isononyl phthalate, di-isodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in the membrane when incorporated is about 0.01% to 20% by weight.

The outer membrane may also contain flux regulating agents. The flux regulating agent is a compound added to assist in regulating the fluid permeability or flux through the membrane. The flux regulating agent can be a flux enhancing agent or a decreasing agent. The agent can be preselected to increase or decrease the liquid flux. Agents that produce a marked increase in permeability to fluid such as water, are often essentially hydrophilic, while those that produce a marked decrease to fluids such as water, are essentially hydrophobic. The amount of regulator in the membrane when incorporated therein generally is from about 0.01% to 20% by weight or more. The flux regulator agents in one embodiment that increase flux include polyhydric alcohols, polyalkylene glycols, polyalkylenediols, polyesters of alkylene glycols, and the like. Typical flux enhancers include polyethylene glycol 300, 400, 600, 1500, 4000, 6000, and the like; glycols (e.g., low molecular weight glycols) such as polypropylene glycol, polybutylene glycol and polyamylene glycol; the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol, and the like; esters such as ethylene glycol dipropionate, ethylene glycol butyrate, butylene glycol dipropionate, glycerol acetate esters, and the like. Representative flux decreasing agents include phthalates substituted with an alkyl or alkoxy or with both an alkyl and alkoxy group such as diethyl phthalate, dimethoxyethyl phthalate, dimethyl phthalate, and di(2-ethylhexyl) phthalate, aryl phthalates such as triphenyl phthalate, and butyl benzyl phthalate; insoluble salts such as calcium sulphate, barium sulphate, calcium phosphate, and the like; insoluble oxides such as titanium oxide; polymers in powder, granule and like form such as polystyrene, polymethylmethacrylate, polycarbonate, and polysulfone; esters such as citric acid esters esterified with long chain alkyl groups; inert and substantially water impermeable fillers; resins compatible with cellulose based wall forming materials, and the like.

Plug 15 fits into the open end 19 of bag 14. Plug 15 is generally cylindrical and is approximately as long as band 22. The exterior of plug 15 forms a fluid tight seal with the portion of the interior surface of bag 14 with which it is in contact. Plug 14 has an axial, central bore 23 extending completely through it. Bore 23 provides access to the interior of bag 14 for filling bag 14 with composition agent 17. Bore 23 is also adapted to receive flow moderator 16. Plug 15 may be made from the same materials as are used to make flexible bag 14; however, the dimensions of plug 15 should be such that it is substantially inflexible.

Flow moderator 16 provides a passageway from the interior of bag 14 to the exterior of dispenser 11 by which composition 17 is discharged from dispenser 11. Flow moderator 16 comprises a conduit, in the form of a rigid cylindrical tube 24, and a head or cap 25. Tube 24 and head 25 may be made from suitable plastics or metals, respectively. The outer diameter of tube 24 is approximately the same as the diameter of bore 23 such that tube 24 may be inserted through bore 23 into bag 14 with tube 24 fitting snugly within bore 23 so as to form an essentially fluid tight seal with plug 15. The length of tube 24 may be such that it extends into bag 14 to at least about 50% of the elongated dimension of the interior of bag 14, i.e., the distance from the inner side of end 18 to the inner end of plug 15. Preferably tube 24 extends into bag 14 over substantially the entire, but not all of (say 85% to 95%), of said elongated dimension. The inner diameter of tube 24 is correlated to the length of tube 24 such that substantial diffusional flow of composition 17 through tube 24 will not occur. Tube 24 is, in effect, a capillary that provides resistance to the flow of composition 17, thereby reducing or eliminating bulk loss of composition 17 from the outlet port of dispenser 10. Although not shown in the drawings, tube 24 may extend outwardly from the exterior of head 25 to provide a site for attachment of a catheter or other dispensing means. Head 25 is preferably hemispherical and may have a diameter approximately equal to the outer diameter of dispenser 10. It also has a diametrical bore 26 for receiving tube 24. As seen in FIG. 2, the flat side of head 25 fits against the top of plug 15 and the top edge of bag 14. Thus, the spherical surface of head 25 provides a smooth, blunt surface that generally aligns with the exterior surface of the bag-solute layer-membrane assembly, which bluntness and alignment are desirable if the dispenser is to be used as an implant to administer drugs to animals or humans.

With reference now to FIG. 5, head 25 has three radial, equispaced grooves 27 in its spherical surface that intersect at bore 23. The outer end of tube 24 is inset slightly from the spherical surface of head 25 (FIG. 2) and thus grooves 27 serve as flow channels for composition 17 exiting from the outer end of tube 24. Grooves 27 are optional.

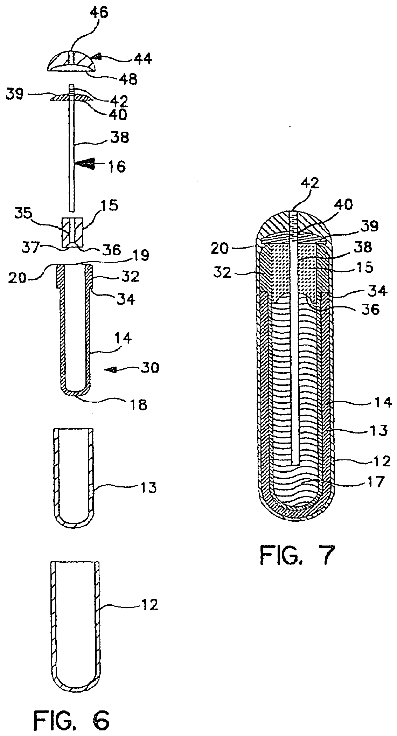

FIGS. 6 and 7 illustrate an osmotic dispenser according to another embodiment of the invention. The dispenser of FIGS. 6 and 7, generally designated 30, is nearly identical to dispenser 10 of FIGS. 1 and 2, and like components are identified with like numerals. Dispenser 30 differs from dispenser 10 in the design of the inner bag 14, the flow modulator 16 and the plug 15, as will now be described.

Bag 14 is elongated and generally cylindrical and is closed at its end 18 and open at its opposite end 19. The edge 20 at open end 19 is a smooth, curved surface, as described above with respect to dispenser 10. The wall of bag 14 is thickened outwardly at 32 to form a shoulder 34. As seen in FIG. 7, the portion of the exterior of bag 14 below shoulder 34 is encapsulated by sleeve 13 whose wall is approximately as thick as shoulder 34 is wide. Sleeve 13 is encapsulated by outer membrane 12. Membrane 12 also covers the exterior of the portion of bag 14 above shoulder 34 and covers the arcuate edge 20 to form a fluid tight seal in this region.

Plug 15 fits into the open end 19 of bag 14. Plug 15 is generally cylindrical and is approximately as long as the thickened portion of bag 14 above shoulder 34. The exterior of plug 15 forms a fluid tight seal with the portion of the interior surface of bag 14 with which it is in contact. Plug 15 has an axial, central bore 35 extending completely through it. Bore 35 provides access to the interior of bag 14 for filling bag 14 with active agent composition 17. Bore 35 is also adapted to receive flow moderator 16. Plug 15 preferably has a hemispherically shaped recess 36 in its inner (bottom) end 37. The presence of such a recess or concavity in end 37 reduces the likelihood of entrapping air in bag 14 when filling bag 14 with composition 17. The plug described with respect to dispenser 10 was generally cylindrical in shape, its inner end joining the wall of the bag at a 90° angle. During the filling of the bag, the fluid has a natural tendency, due to its high surface tension, to form a curved surface beginning near the top of the wall of the bag and continuing up to the filling/discharge port. This curvature causes an air pocket at the intersection of the wall of the bag and the plug. The plug of dispenser 30 has a hemispherically recessed lower surface, curved to substantially match the arc created by the surface tension of the drug solution during the filling process. This reduces the volume of the dispenser that cannot be filled due to air entrapment from approximately 15% to less than 2% to 3%.

Flow moderator 16 provides the passageway from the interior of bag 14 to the exterior of the dispenser 30 by which composition 17 is discharged from dispenser 30. Flow moderator 16 comprises a conduit, in the form of a rigid cylindrical tube 38, and a dome-shaped head 39. Tube 38 and head 39 may be made from suitable plastics or metals. Head 39 has an axial, threaded bore 40 that receives threaded end 42 of tube 38. As shown in FIGS. 6 and 7, end 42 extends outwardly from the spherical surface of head 39 to provide a site for attaching an external catheter tube (not shown) in the event dispenser 30 is to be used to administer composition 17 to a remote location. The outer diameter of tube 38 is approximately the same as the diameter of bore 35 such that tube 38 may be inserted through bore 35 into bag 14 with tube 38 fitting snugly within bore 35 so as to form an essentially fluid tight seal with plug 15. Head 39 has a diameter slightly larger than the outer diameter of plug 15, and, as seen in FIG. 7, the flat side of head 39 fits against the top of plug 15.

Dispenser 30 also can include a flow moderator cap 44 which may be used to cover protruding end 42 of tube 38 when dispenser 30 is used without an external catheter tube connection. Cap 44 is crescent-shaped and has an axial threaded bore 46 that receives end 42. The curvature of its concave underside 48 matches the convexity of the top surface of head 39 so that the former fits tightly against the latter, as seen in FIG. 7. The outer diameter of cap 44 is the same as the outer diameter of membrane 12. Thus the hemispherical exterior of cap 44 provides a smooth, blunt surface that aligns with the exterior surface of membrane 12.

The dispensers of the present invention may be made by any of the various methods and techniques known and available to those skilled in the art.

For instance, the components of the above-described dispensers may be made and assembled as follows. The inner, flexible bag may be injection molded from suitable polymer compositions by known techniques. The bag may then be placed on a support means, such as a mandrel, and a suspension of the osmotically effective solute in an appropriate suspending medium is prepared. The supported bag may then be dipped repeatedly in the suspension to the desired depth, with intervening drying periods, until a layer of osmotic solute of desired thickness is formed.

A solution of membrane material may then be made, and the supported, solute coated bag may be dipped repeatedly into the solution to a depth just above the top edge of bag 14, with intervening drying periods, until an outer membrane of desired thickness is formed. The outer membrane may be prepared from cellulose-based polymers, such as cellulose acetate butyrate, cellulose acetate propionate and cellulose triacetate. Other polymers that may be used include acrylic polymers, such a polymethylmethacryate. The polymers may be used alone or as mixtures with each other. Ethyl cellulose may also be added to the polymers or mixtures of the polymers to provide presently preferred polymeric bases for the membrane coatings. The mixtures may contain polymer to ethyl cellulose in ratios generally in the range of 1:9 to 9:1, more often 1:5 to 5:1. Representative examples are those mixtures comprised of 1 part of polymethylmethacrylate to 1 part of ethyl cellulose, 1 part of polymethylmethacrylate to 3 parts of ethyl cellulose, and 5 parts of polymethylmethacrylate to 4 parts of ethyl cellulose. Cellulose polymers are soluble in various solvents in varying degrees. For example, cellulose acetate butyrate and cellulose triacetate are readily soluble in methylene chloride. However, use of this solvent is not preferred for regulatory and safety reasons. The celluloses are soluble in acetone, ethyl acetate, methyl acetate, mixtures of methylene chloride and isopropyl alcohol, mixtures of acetone and water, and mixtures of methyl and ethyl alcohol and ethyl acetate. Preferred solvents are acetone, acetone and water, and acetone and lower alkanols having 1-8 carbon atoms. Representative alcohols include methanol, ethanol, isopropyl alcohol, and the like.

Although methylene chloride is not a preferred solvent for reasons of safety, devices having an outer membrane of a cellulose-based polymer prepared from solvents other than methylene-chloride may be prone to development of fissures at sharp edge regions of the devices described herein. In accord with one aspect of this invention, however, it has been surprisingly discovered that such problems can be avoided when the open end of the inner bag and/or the cap adapted for sealing the open end are characterized by an arcuate edge. In this manner, satisfactory devices such as those described herein having an outer membrane of a cellulose-based or acrylic polymer prepared from solvents other than methylene-chloride, such as acetone and acetone-based solvent systems, can be prepared.

After the outer membrane is formed, the mandrel may then be removed and a plug, which may be injection molded by known techniques, may be glued into the open end of the bag. A head may be machined by known techniques if it is metal, or injection molded by known techniques if made from a synthetic polymer. The end of the capillary tube may be affixed within the bore by press-fitting, gluing, or other known techniques.

Alternatively, the plug may be glued into the open end of the bag prior to the solution of membrane material being coated upon the surface of the sealed inner bag and thereby encapsulating the polymer composition. Coating may be accomplished by known methods such as pan coating and fluidized spray coating.

Preferred membranes are those formed from cellulose acetate butyrate, cellulose acetate propionate, and polymethylacrylate, either alone or in mixtures with each other and optionally ethyl cellulose, and that are deposited out of acetone-based solvent systems. It has also been discovered that polymer-solvent compositions that are useful to prepare outer membranes for the devices described herein also have utility with respect to other osmotic devices and systems generally that utilize a semipermeable membrane. Representative osmotic devices and systems have been described, for example, in U.S. Pat. Nos. 4,503,030; 4,519,801; 4,522,625; 4,553,973; 4,576,604; 4,578,075; 4,627,850; 4,777,049; 4,783,337; 4,863,744; 4,940,465; 4,946,687; 4,948,592; 5,019,396; 5,208,037; 5,232,705; 5,324,280; and 5,413,572.

The cellulose acetate butyrate, cellulose acetate propionate and polymethylmethacryate may be used alone or as mixtures with each other. Ethyl cellulose may also be added to the polymers or mixtures of the polymers to provide presently preferred polymeric bases for the membranes that are formed by depositing the polymers on the osmotic systems from compositions of the polymer and solvent. Preferred solvents are acetone, acetone and water, and acetone and lower alkanols having 1-8 carbon atoms. Representative alcohols include methanol, ethanol, isopropyl alcohol, and the like. Optionally, one or more additives selected from the group consisting of plasticizers and flux enhancers, such as those described previously herein may be added to the coating compositions. The lower alkanols may be straight or branched chain, and are illustrated by representative alcohols such as methanol, ethanol, isopropyl alcohol, and the like. Typically, the concentration of acetone in the solvent is at least 80% by volume, more often about 90%. The polymer concentration in the solvent is between 1-15% (w/v), and may range from 1% to 10% (w/v), often 1% to 5% (w/v). The coating compositions typically provide for formed semipermeable membrane coats exhibiting a water transmission rate (i.e., Kπ x 10⁻³) of between 1-60 cc·mil/cm²·hr, often 3-40 cc·mil/cm²·hr, as measured by standard methods using sodium chloride. The coats may be deposited on the osmotic cores by conventional methods, e.g., pan coating. For the particular devices described herein, the coat may be from 10-80% by weight of the bag coated with the osmotically-effective solute. For more general osmotic systems such as those described in the aforesaid patents, coatings of 1-50%, more often 1-30%, by weight of the weight of the bilayer drug/push composition cores is typical. For the general osmotic drug systems adapted for once-a-day administration, the coating compositions used in this invention provide dosage forms with coated outer membranes that have 0-4 hour start up periods and permit about 90% of the drug to be delivered over 20 or more hours. For sustained release dosage forms intended for a twice-a-day administration, the membranes formed from the coating compositions used in the invention allow for start-up times of 0-2 hours.

Particularly useful polymers for preparing the coating compositions are the cellulose acetate butyrate polymers having an acetyl content ranging from about 2%-30%, a butyryl content of about 17%-52% and a hydroxyl content of about 1%-5%, with number average molecular weights ranging from 20,000-60,000. Such polymers are available from the Eastman Chemical Company, Kingsport, Tenn., under the grade designations CAB-171, -321, -381, -500, -531, -551, and -553. Cellulose acetate propionates are available from the same company having acetyl content of 0.6%-2.5%, propionyl content of 42.5-46%, and hydroxyl content of 1.8-5%, and number average molecular weights of 15,000-75,000. Grade designations are CAP-482 and -504. The polymethylmethacrylates may include, for example, those sold under the trade name Plexiglas^{®}, particularly Plexiglas^{®} V-825 sold by PolyOne Corporation (previously M.A. Hanna Company), Lemont, Ill., USA, and Rohm and Haas, Philadelphia, Pa., USA. Ethyl cellulose may be obtained from a number of suppliers to the pharmaceutical industry, including Dow Chemical Company under the trade name Ethocel^{™}. Representative pharmaceutical grades may have viscosities in the range of 3-110 cP (measured as 5% solutions at 25 degrees Centigrade in an Ubbelohde viscometer with a solvent of 80% toluene and 20% alcohol) and an ethoxyl content of 48%-49.5%. The foregoing examples are illustrative and those skilled in the pharmaceutical formulation and manufacturing arts may select materials having different physical properties for a particular application.

The coating compositions may optionally include plasticizers and flux regulating agents such as described previously.

The coating compositions are prepared by conventional procedures. Polymer is mixed, along with any optional additives, with the selected solvent system and allowed to dissolve to form a coating solution. That solution is then applied to the osmotic systems by pan coating or the like. The coated systems typically are allowed to dry or dried under forced conditions to allow for formation of the solid, semipermeable membrane encapsulating the osmotic system or device.

The antioxidant used to make the dispenser of the present invention may be in the form of particles. The antioxidant may be ground into a fine powder and screened, e.g., through a 30 mesh sieve. The particles may have an average particle size ranging from 0.1 µm to 500 µm, e.g., 1 µm to 300 µm, 5 µm to 200 µm, or 10 µm to 100 µm. The particles may have a particle size of less than 600 µm.

The antioxidant particles may be melt processed (e.g., extruded, compression molded, or injection molded) with other components used to form the solute composition. The melt processing temperature may range from 150°C to 250°C, such as 160°C to 220°C or 170°C to 200°C.

As noted above, the dispensers may be sterilized. As used herein, "sterile" refers to the composition meeting the requirements of sterility enforced by medicine regulatory authorities, such as the MCA in the UK or the FDA in the US. Tests are included in current versions of the compendia, such as the British Pharmacopoeia and the US Pharmacopoeia. For instance, the dispenser in question may be essentially free of viable micro-organisms according to Ph. Eur. 2.6. For example, the terminally sterilized dispenser may have a probability of nonsterility (PNS) of not more than one in a million units produced. This is often stated as a PNS of 10⁻⁶, or the probability of product bioburden surviving the sterilization process in any single unit of product is less than one in one million.

The dispensers may be sterilized prior to use using any suitable method known in the art, e.g., sterilization with an ionizing radiation such as gamma irradiation, e-beam radiation or x-ray radiation, at a dose of about 10 kGy to about 25kGy.

Suitable forms of ionizing radiation include, e.g., gamma radiation, e-beam radiation and x-ray radiation. One of ordinary skill in the art will be able to determine an appropriate sterilizing dose of radiation based on a variety of factors including, e.g., the type of radiation, the shape, size, and/or composition of the material to be sterilized, the desired level of sterility and the amount of contamination present prior to sterilization. The irradiation may be conducted at from about 0°C to about 30°C, e.g., from about 0°C to about 5°C, from about 5°C to about 10°C, from about 10°C to about 15°C, from about 15°C to about 20°C, from about 20°C to about 25°C, or from about 25°C to about 30°C.

In some embodiments, a suitable dose of sterilizing radiation is a dose of about 10 kGy to about 35 kGy, e.g., about 15 kGy to about 25 kGy or about 15 kGy to about 20 kGy.

The dispensers may be filled with fluid via the bore in the plug. For instance, the needle of a fluid loaded syringe may be inserted through the bore and the syringe's contents discharged into the bag. To ensure that a predetermined fluid pumping rate is achieved, it is desirable to completely fill bag with fluid. After the bag is filled, the tube of the flow moderator may be inserted through the bore to the position shown in the figures. The tube functions as a capillary and inhibits loss of fluid from the dispensers even though they are subjected to substantial movement or tipped upside down.

The dispensers operate in the following manner. Once placed in an aqueous environment, such as within a body cavity or within body tissue, water from the environment is imbibed by the osmotic layer through the outer membrane at a rate determined by the osmotic activity of the osmotically effective solute, and the osmotic reflection coefficient, composition, thickness, and area of the outer membrane. The imbibed water causes the volume of the space between the inner surface of the outer membrane and the exterior of the inner bag (the space initially occupied by the osmotic layer) to increase. And since the outer membrane may be shape-retaining, the imbibed water generates hydraulic pressure on the exterior of the fluid bag causing the bag to be squeezed inwardly. This squeezing forces fluid through the tube and out of the dispenser. Any air bubbles that were trapped within the bag during filling will tend to be located adjacent to the inner end of the plug or the inner surface of the bag, depending on the attitude of the dispenser. Therefore, these air bubbles are not likely to block the entrance to tube and interrupt or impede the flow of fluid therethrough.

As noted above, the dispensers may include an active agent. A variety of active agents may be delivered using the dispensers disclosed herein. General classes of active agents which may be delivered include, for example, proteins, peptides, nucleic acids, nucleotides, nucleosides and analogues thereof, antigens, antibodies, and vaccines; as well as low molecular weight compounds.

In some embodiments, the active agent has a molecular weight from about 200 D to about 1000 kD, from about 10 kD to about 150 kD, from about 20 kD to about 100 kD, from about 30 kD to about 80 kD, from about 40 kD to about 70 kD, or from about 50 kD to about 60 kD.

In some embodiments, the active agent comprises particles. The particles may have an average particle size ranging from 1 nm to 250 µm, e.g., 10 nm to 100 µm, 100 nm to 50 µm, or 1 µm to 10 µm.

Active agents which may be delivered using the dispensers disclosed herein include, but are not limited to, agents which act on the peripheral nerves, adrenergic receptors, cholinergic receptors, the skeletal muscles, the cardiovascular system, smooth muscles, the blood circulatory system, synaptic sites, neuroeffector junction sites, endocrine and hormone systems, the immunological system, the reproductive system, the skeletal system, autacoid systems, the alimentary and excretory systems, the histamine system and the central nervous system.

Suitable active agents may be selected, for example, from chemotherapeutic agents, epigenetic agents, proteasome inhibitors, adjuvant drugs, anti-emetics, appetite stimulants, anti-wasting agents and high potency opioids.

Suitable active agents may also be selected, for example, from antineoplastic agents, cardiovascular agents, renal agents, gastrointestinal agents, rheumatologic agents and neurological agents among others.

### Protein, Polypeptides and Peptides as Active Agents

Proteins useful in the disclosed dispensers may include, for example, molecules such as cytokines and their receptors, as well as chimeric proteins comprising cytokines or their receptors, including, for example tumor necrosis factor alpha and beta, their receptors and their derivatives; renin; growth hormones, including human growth hormone, bovine growth hormone, methione-human growth hormone, des-phenylalanine human growth hormone, and porcine growth hormone; growth hormone releasing factor (GRF); parathyroid and pituitary hormones; thyroid stimulating hormone; human pancreas hormone releasing factor; lipoproteins; colchicine; prolactin; corticotrophin (adrenocorticotropic hormone (ACTH)); thyrotropic hormone; oxytocin; vasopressin; somatostatin; somatostatin analogs; octreotide; lypressin; pancreozymin; leuprolide; alpha-1-antitrypsin; insulin A-chain; insulin B-chain; proinsulin; follicle stimulating hormone; calcitonin; luteinizing hormone; luteinizing hormone releasing hormone (LHRH); LHRH agonists and antagonists; glucagon; clotting factors such as factor VIIIC, factor IX, tissue factor, and von Willebrands factor; anti-clotting factors such as Protein C; atrial natriuretic factor; lung surfactant; a plasminogen activator other than a tissue-type plasminogen activator (t-PA), for example a urokinase; bombesin; thrombin; hemopoietic growth factor; enkephalinase; RANTES (regulated on activation normally T-cell expressed and secreted); human macrophage inflammatory protein (MIP-1-alpha); a serum albumin such as human serum albumin; mullerian-inhibiting substance; relaxin A-chain; relaxin B-chain; prorelaxin; mouse gonadotropin-associated peptide; chorionic gonadotropin; gonadotropin releasing hormone; bovine somatotropin; porcine somatotropin; a microbial protein, such as beta-lactamase; DNase; inhibin; activin; vascular endothelial growth factor (VEGF); receptors for hormones or growth factors; integrin; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); fibroblast growth factor such as acidic FGF and basic FGF; epidermal growth factor (EGF); transforming growth factor (TGF) such as TGF-alpha and TGF-beta, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins; CD proteins such as CD-3, CD-4, CD-8, and CD-19; erythropoietin; osteoinductive factors; immunotoxins; a bone morphogenetic protein (BMP); an interferon such as interferon-alpha (e.g., interferonα2A or interferonα2B ), -beta, -gamma, -lambda and consensus interferon; colony stimulating factors (CSFs), e.g., M-CSF, GM-CSF, and G-CSF; interleukins (ILs), e.g., IL-1 to IL-10; superoxide dismutase; T-cell receptors; surface membrane proteins; decay accelerating factor; viral antigen such as, for example, a portion of the HIV-1 envelope glycoprotein, gp120, gp160 or fragments thereof; transport proteins; homing receptors; addressins; fertility inhibitors such as the prostaglandins; fertility promoters; regulatory proteins; antibodies and chimeric proteins, such as immunoadhesins; precursors, derivatives, prodrugs and analogues of these compounds, and pharmaceutically acceptable salts of these compounds, or their precursors, derivatives, prodrugs and analogues.

Suitable proteins or peptides may be native or recombinant and include, e.g., fusion proteins.

In some embodiments, the protein is a growth hormone, such as human growth hormone (hGH), recombinant human growth hormone (rhGH), bovine growth hormone, methione-human growth hormone, des-phenylalanine human growth hormone, and porcine growth hormone; insulin, insulin A-chain, insulin B-chain, and proinsulin; or a growth factor, such as vascular endothelial growth factor (VEGF), nerve growth factor (NGF), platelet-derived growth factor (PDGF), fibroblast growth factor (FGF), epidermal growth factor (EGF), transforming growth factor (TGF), and insulin-like growth factor-I and -II (IGF-I and IGF-II).

Suitable peptides for use as the active agent in the dispensers disclosed herein include, but are not limited to, glucagon-like peptide-1 (GLP-1) and precursors, derivatives, prodrugs and analogues thereof. In some embodiments, a suitable peptide is a GLP-1 receptor agonist, e.g., exenatide or liraglutide.

In addition, a suitable protein, polypeptide, peptide; or precursor, derivative, prodrug or analogue thereof is one which is capable of forming an insoluble component comprising active agent, e.g., an insoluble active agent complex, e.g., by complexing with a metal or other precipitating and/or stabilizing agent.

### Nucleic Acids as Active Agents

Nucleic acid active agents include nucleic acids as well as precursors, derivatives, prodrugs and analogues thereof, e.g., therapeutic nucleotides, nucleosides and analogues thereof; therapeutic oligonucleotides; and therapeutic polynucleotides. Active agents selected from this group may find particular use as anticancer agents and antivirals. Suitable nucleic acid active agents may include for example ribozymes, antisense oligodeoxynucleotides, aptamers, and siRNA. Examples of suitable nucleoside analogues include, but are not limited to, cytarabine (araCTP), gemcitabine (dFdCTP), and floxuridine (FdUTP).

### Other Active Agent Compounds

A variety of other active agent compounds may be used in the compositions disclosed herein. Suitable compounds may include, but are not limited to, compounds directed to one or more of the following drug targets: Kringle domain, Carboxypeptidase, Carboxylic ester hydrolases, Glycosylases, Rhodopsin-like dopamine receptors, Rhodopsin-like adrenoceptors, Rhodopsin-like histamine receptors, Rhodopsin-like serotonin receptors, Rhodopsin-like short peptide receptors, Rhodopsin-like acetylcholine receptors, Rhodopsin-like nucleotide-like receptors, Rhodopsin-like lipid-like ligand receptors, Rhodopsin-like melatonin receptors, Metalloprotease, Transporter ATPase, Carboxylic ester hydrolases, Peroxidase, Lipoxygenase, DOPA decarboxylase, A/G cyclase, Methyltransferases, Sulphonylurea receptors, other transporters (e.g., Dopamine transporter, GABA transporter 1, Norepinephrine transporter, Potassium-transporting ATPase α-chain 1, Sodium-(potassium)-chloride cotransporter 2, Serotonin transporter, Synaptic vesicular amine transporter, and Thiazide-sensitive sodium-chloride cotransporter), Electrochemical nucleoside transporter, Voltage-gated ion channels, GABA receptors (Cys-Loop), Acetylcholine receptors (Cys-Loop), NMDA receptors, 5-HT3 receptors (Cys-Loop), Ligand-gated ion channels Glu: kainite, AMPA Glu receptors, Acid-sensing ion channels, Ryanodine receptors, Vitamin K epoxide reductase, MetGluR-like GABAs receptors, Inwardly rectifying K⁺ channel, NPC1L1, MetGluR-like calcium-sensing receptors, Aldehyde dehydrogenases, Tyrosine 3-hydroxylase, Aldose reductase, Xanthine dehydrogenase, Ribonucleoside reductase, Dihydrofolate reductase, IMP dehydrogenase, Thioredoxin reductase, Dioxygenase, Inositol monophosphatase, Phosphodiesterases, Adenosine deaminase, Peptidylprolyl isomerases, Thymidylate synthase, Aminotransferases, Farnesyl diphosphate synthase, Protein kinases, Carbonic anhydrase, Tubulins, Troponin, Inhibitor of IκB kinase-β, Amine oxidases, Cyclooxygenases, Cytochrome P450s, Thyroxine 5-deiodinase, Steroid dehydrogenase, HMG-CoA reductase, Steroid reductases, Dihydroorotate oxidase, Epoxide hydrolase, Transporter ATPase, Translocator, Glycosyltransferases, Nuclear receptors NR3 receptors, Nuclear receptors: NR1 receptors, and Topoisomerase.

In some embodiments, the active agent is a compound targeting one of rhodopsin-like GPCRs, nuclear receptors, ligand-gated ion channels, voltage-gated ion channels, penicillin-binding protein, myeloperoxidase-like, sodium: neurotransmitter symporter family, type II DNA topoisomerase, fibronectin type III, and cytochrome P450.

In some embodiments, the active agent comprises at least one member selected from adrenocorticotropic hormone (ACTH), AIDS/HIV drug, aldosterone, amphetamine, analgesic, angiotensin, antibiotic, antibody, anticonvulsive agent, antidepressant, antihypertensive, antipsychotic, antisense oligonucleotide, barbiturate, benzodiazepine, bromodeoxyuridine, cannabinoid, calcitonin, calcitrol, chemotherapeutic agent, cholinergic agent, cocaine, corticosterone, cortisol, cyclosporine, cytokine, deoxycorticosterone, dexamethasone, dopamine, dopaminergic agent, enzyme inhibitor, epinephrine, erythropoietin, estrogen, follicle stimulating hormone, gamma-aminobutyric acid (GABA), growth factor, growth hormone, immunosuppressant, insulin, leptin, lipopolysaccharide, luteinizing hormone, luteinizing-hormone-releasing hormone (LHRH), metal, metalloproteinase inhibitor, naloxone, naltrexone, neurotrophic factor, nicotine, nitric oxide, N-methyl-D-aspartate (NMDA) agonist, NMDA antagonist, norepinephrine, NSAID, opioid, opiate peptide, parathyroid hormone, progesterone, prolactin, prostaglandin, siRNA, testosterone, thyroid hormone, thyroxine (T4), triiodothyronine (T3), vascular endothelial factor, vasopressin, and vitamin.

In some embodiments, the active agent is an anticancer agent. Suitable anticancer agents include, but are not limited to, Actinomycin D, Alemtuzumab, Allopurinol sodium, Amifostine, Amsacrine, Anastrozole, Ara-CMP, Asparaginase, Azacytadine, Bendamustine, Bevacizumab, Bicalutimide, Bleomycin (e.g., Bleomycin A₂ and B₂), Bortezomib, Busulfan, Camptothecin sodium salt, Capecitabine, Carboplatin, Carmustine, Cetuximab, Chlorambucil, Cisplatin, Cladribine, Clofarabine, Cyclophosphamide, Cytarabine, Dacarbazine, Dactinomycin, Daunorubicin, Daunorubicin liposomal, Dacarbazine, Decitabine, Docetaxel, Doxorubicin, Doxorubicin liposomal, Epirubicin, Estramustine, Etoposide, Etoposide phosphate, Exemestane, Floxuridine, Fludarabine, Fluadarabine phosphate, 5-Fluorouracil, Fotemustine, Fulvestrant, Gemcitabine, Goserelin, Hexamethylmelamine, Hydroxyurea, Idarubicin, Ifosfamide, Imatinib, Irinotecan, Ixabepilone, Lapatinib, Letrozole, Leuprolide acetate, Lomustine, Mechlorethamine, Melphalan, 6-Mercaptopurine, Methotrexate, Mithramycin, Mitomycin C, Mitotane, Mitoxantrone, Nimustine, Ofatumumab, Oxaliplatin, Paclitaxel, Panitumumab, Pegaspargase, Pemetrexed, Pentostatin, Pertuzumab, Picoplatin, Pipobroman, Plerixafor, Procarbazine, Raltitrexed, Rituximab, Streptozocin, Temozolomide, Teniposide, 6-Thioguanine, Thiotepa, Topotecan, Trastuzumab, Treosulfan, Triethylenemelamine, Trimetrexate, Uracil Nitrogen Mustard, Valrubicin, Vinblastine, Vincristine, Vindesine, Vinorelbine, and analogues, precursors, derivatives and pro-drugs thereof. It should be noted that two or more of the above compounds may be used in combination.

Active agents of interest for use in the disclosed dispensers may also include opioids and derivatives thereof as well as opioid receptor agonists and antagonists, e.g., methadone, naltrexone, naloxone, nalbuphine, fentanyl, sufentanil, oxycodone, oxymorphone, hydrocodone, hydromorphone, and pharmaceutically acceptable salts and derivatives thereof.

In other embodiments, the active agent is used to establish an animal model of a human disease. Examples of these active agents include, but are not limited to, endotoxin / LPS, beta amyloid, MPTP & MPP⁺, rotenone, and etrodotoxin.

The present invention will be further illustrated by way of the following Examples. These examples are non-limiting and do not restrict the scope of the invention. Unless stated otherwise, all percentages, parts, etc. presented in the examples are by weight.

### EXAMPLES

### COMPARATIVE EXAMPLE 1

### OBJECTIVE

The present Comparative Example involves determining if any formaldehyde released from ALZET^{®} samples, which were immersed in water and incubated at 37°C for 14 days prior to test.

Two studies are described here. In Study I, the finished product ALZET^{®} pump was tested as well as the Kraton^{™} copolymer beads. In Study II, ALZET^{®} parts including reservoir, salt pellets (used to form solute layers / salt sleeves), Kraton^{™} copolymer beads (used to form flexible containers / bags), plug, and salt sleeves were examined.

### STUDY I

### MATERIALS

**ALZET^{®} pumps**
**Kraton^{™} copolymer beads used to form reservoir of ALZET^{®} pumps**
**ALZET^{®} 200X Filling Tube**
**ALZET^{®} Flow Moderators**
**1 mL EXEL Syringes**
**20 ml Scintillation Glass Vials**
**Milli Q Water**
**Formaldehyde Test Kit, EM Quant**

### EXPERIMENTAL

1. The weight of an ALZET^{®} pump with a flow moderator was tared, and the pump was filled with Milli Q water using a fill tube attached to a 1 mL syringe (three ALZET^{®} pumps each). The flow moderator was inserted into the pump.
2. The pump containing the water with the flow moderator was weighed.
3. In each of two Scintillation vials, seventeen Kraton^{™} copolymer beads used to make the containers of ALZET^{®} pumps were placed along with 1 mL Milli Q water. The gross weight was recorded.
4. All the samples as well as a Scintillation vial containing about 15 mL Milli Q water were placed into an incubator at 37°C and shaken at 100 rpm for 14 days.
5. The gross weight of all samples was weighed after 14 days incubation.
6. The water from the pumps (3 each in one lot) was removed and combined into a 2 mL test tube. The total volume was 0.45 mL in each lot. One drop of formaldehyde test reagent FO-1 was added followed by mixing.
7. 0.5 mL of water was withdrawn from the vial containing Kraton^{™} copolymer beads and dispensed into a 2 mL test tube. One drop of formaldehyde test reagent FO-1 was added followed by mixing.
8. 0.5 mL of standard formaldehyde solutions, 10 mg/L and 20 mg/L respectively, was dispensed into 2 mL test tubes, and one drop of reagent FO-1 was added into each tube followed by mixing.
9. The samples were tested using the test strips, and the color of the strips was compared. The corresponding result in mg/L HCHO was determined.
10. The formaldehyde test was repeated on ALZET^{®} pumps (experiment was performed 7 weeks later).

### RESULTS

### HCHO Test in ALZET^{®} Samples

| Sample | Water filled before incubation (g) | Water remaining after incubation (g) | Strip color in test | Note |
|---|---|---|---|---|
| Pumps | 0.229 | 0.172 | Pink | No HCHO |
| | 0.226 | 0.141 | | |
| | 0.229 | 0.175 | | |
| Pumps | 0.228 | 0.136 | Brown | Strip color is close to the one from 10 mg/L HCHO solution |
| | 0.224 | 0.148 | | |
| | 0.225 | 0.129 | | |
| Kraton^{™} Copolymer Beads | 1.005 | 1.000 | Pink | No HCHO |
| | 1.003 | 0.994 | Pink | No HCHO |
| Incubated Milli Q Water | | | Pink | No HCHO |
| Fresh Milli Q Water | | | Pink | No HCHO |
| 10 mg/L HCHO Solution | | | Brown | |
| 20 mg/L HCHO Solution | | | Dark Brown | |

### Repeat HCHO Test in ALZET^{®} Pumps

| Sample | Strip color in test | Note |
|---|---|---|
| Pumps | Brown | Strip color is close to the one from 10 mg/L HCHO solution |
| Pumps | Brown | Strip color is close to the one from 10 mg/L HCHO solution |
| Incubated Milli Q Water | Pink | No HCHO |
| Fresh Milli Q Water | Pink | No HCHO |
| 10 mg/L HCHO Solution | Brown | |
| 20 mg/L HCHO Solution | Dark Brown | |

### SUMMARY

The color of strips from the pump sample solutions was close to the 10 mg/L formaldehyde solution suggesting HCHO was released from the pumps after 14 day incubation in the water, and the concentration of the HCHO was approximately 10 mg/L. About 0.0015 mg of HCHO was released from each pump.

### STUDY II

### MATERIALS

**ALZET^{®} reservoirs: 2 each in one 20 ml Scintillation glass vial**
**ALZET^{®} salt pellets (NaCl 45 wt%, PolyOx^{™} 40 wt% and PEG 8000 15 wt%): 10 each in one 20 ml Scintillation glass vials, two vials total**
**Kraton^{™} copolymer beads: 18 each 20 ml Scintillation glass vials, two vials total**
**ALZET^{®} plugs, 3 each in one 20 ml Scintillation glass vial, one vial total**
**ALZET^{®} salt sleeves, 2 each in one 20 ml Scintillation glass vial, one vial total**
**20 ml Scintillation Glass Vials**
**Milli Q Water**
**Formaldehyde Test Kit, EM Quant**

### EXPERIMENTAL

1. Milli Q water was dispensed into each vial: 2.5 mL into vials containing ALZET^{®} reservoirs, salt sleeves or salt pellets such that the reservoirs were fully filled with the water; 2.0 mL and 1.5 mL water was dispensed into the vials containing ALZET^{®} plugs and Kraton^{™} copolymer beads, respectively.
2. The samples as well as Scintillation vial containing about 15 mL Milli Q water were placed into an incubator at 37°C and shaken at 100 rpm for 14 days.
3. The gross weight of all samples was weighed after 14 days incubation.
4. 0.5 mL water was withdrawn from incubated vials and dispensed into a 2 mL test tube. One drop of formaldehyde test reagent FO-1 was added followed by mixing.
5. 0.5 mL of standard formaldehyde solutions, 10 mg/L and 20 mg/L respectively, was dispensed into 2 mL test tubes, and one drop of reagent FO-1 was added into each tube followed by mixing.
6. The samples were tested using the test strips, and the color of the strips was compared. The corresponding result in mg/L HCHO was determined.

### RESULTS

### HCHO Test in ALZET^{®} Samples

| Sample | Strip color in test | Note |
|---|---|---|
| Reservoirs | Pink | No HCHO |
| Salt pellets | NA | Salt pellets were dissolved in the water and gave a gel-like mixture; test could not be performed |
| Salt Sleeves | Dark Brown | Strip color is close or even darker in comparison with the one from 20mg/L HCHO solution |
| Plugs | Pink | No HCHO |
| Kraton^{™} Copolymer Beads | Pink | No HCHO |
| Incubated Milli Q Water | Pink | No HCHO |
| Fresh Milli Q Water | Pink | No HCHO |
| 10 mg/L HCHO Solution | B row n | |
| 20 mg/L HCHO Solution | Dark Brown | |

### SUMMARY

The salt sleeves were dissolved in the water, and the color of strips in the testing on the salt sleeve sample was close or even darker in comparison with the 20 mg/L formaldehyde solution, which suggested HCHO was detected and the concentration of the HCHO was approximately 20 mg/L. The salt pellets were dissolved in the water to give a gel-like mixture, and the test could not be performed. The color of strips from the samples of reservoirs, Kraton^{™} copolymer beads, and plugs were pink and were the same as the ones from the fresh Milli Q water and incubated Milli Q water, which indicated no HCHO was detected or if there was HCHO released, the concentration of HCHO was less than 10 mg/L.

### COMPARATIVE EXAMPLE 2

### OBJECTIVE

The present Comparative Example involves determining if any formaldehyde released from ALZET^{®} salt pellet solution through ALZET^{®} reservoir into water after incubation at 37°C for 14 days.

Study I was to confirm the presence of HCHO in the salt pellet aqueous solution. The HCHO release from the salt pellet solution through the reservoir into water was described in Study II.

### STUDY I (Formaldehyde Test in ALZETO Salt Pellet Aqueous Solution)

### MATERIALS

**ALZET^{®} salt pellets (NaCl 45 wt%, PolyOx^{™} 40 wt% and PEG 8000 15 wt%): 3 each in one 20 ml Scintillation glass vial, one vial total**
**20 ml Scintillation Glass Vials**
**Milli Q Water**
**Formaldehyde standard solutions:**
   **HCHO concentrations were 10 mg/L, 20 mg/L, 40 mg/L and 60 mg/L**
**Formaldehyde Test Kit, EM Quant**

### EXPERIMENTAL

1. The glass vial was tared, and 3 salt pellets were placed in it. The salt pellets were weighed: 0.875 g / 3 salt pellets, each salt pellet weighed 0.292 g.
2. 10 mL of Milli Q water was added to the vial on a rotator for mixing. After mixing overnight, most of the salt pellets were dissolved. Some precipitate was found.
3. 0.5 mL of the clear solution was withdrawn from the vial and dispensed into a 2 mL test tube. One drop of formaldehyde test reagent FO-1 was added followed by mixing.
4. 0.5 mL Milli Q water was dispensed into a 2 mL test tube. One drop of formaldehyde test reagent FO-1 was added followed by mixing.
5. 0.5 mL of standard formaldehyde solutions, 10 mg/L, 20 mg/L, 40 mg/L and 60 mg/L, respectively, was dispensed into 2 mL test tubes, and one drop of reagent FO-1 was added into each tube followed by mixing.
6. The samples were tested using the test strips, and the color of the strips was compared. The corresponding result in mg/L HCHO was determined.

### RESULTS

### HCHO Test in ALZET^{®} Samples

| Sample | Wt of pellets (g) | Water added (mL) | Strip color in test | Note |
|---|---|---|---|---|
| Salt Pellets | 0.875 | 10 | Dark Brown | Color of strip was darker than the one from 20 mg/L HCHO solution and lighter than the one from 40 mg/L HCHO solution |
| Incubated Milli Q Water | | | Pink | No HCHO |
| Fresh Milli Q Water | | | Pink | No HCHO |
| 10 mg/L HCHO Solution | | | Brown | |
| 20 mg/L HCHO Solution | | | Dark Brown | |
| 40 mg/L HCHO Solution | | | Dark Brown | |

### SUMMARY

The color of strips in the testing on the salt pellet solution was darker than the one from 20 mg/L HCHO solution and lighter than 40 mg/L HCHO solution suggesting HCHO concentration was in the range of 20 mg/L to 40 mg/L.

### STUDY II (PERMEATION STUDY OF HCHO THROUGH ALZET^{®} RESERVOIR INTO WATER)

### MATERIALS

**ALZET^{®} reservoirs**
**ALZET^{®} pellet solution: 3 salt pellets dissolved in 10 mL Milli Q water**
**Cyclohexane Glue (Kraton^{™} copolymer and cyclohexane)**
**ALZET^{®} 200X Filling Tube**
**Milli Q Water**
**Formaldehyde Test Kit, EM Quant**

### EXPERIMENTAL

1. Plug was secured in the mouth of the reservoir using cyclohexane glue and air dried at R.T. for 24 hours prior to use (6 reservoirs total).
2. About 0.2-0.25 mL of salt pellet solution was injected into each reservoir through the plug using a 1 mL syringe equipped with a filling tube.
3. The reservoirs were placed into 2 mL plastic test tubes, and Milli Q water was added into the test tubes to the level about 1-2 mm below the top of the reservoirs (about 0.5 mL).
4. Empty reservoirs (3 reservoirs total) were placed into 2 mL plastic test tubes, and Milli Q water was added into the test tubes to the level about 1-2 mm below the top of the reservoirs (about 0.5 mL).
5. Milli Q water was dispensed into 2 mL plastic test tubes and 20 mL glass Scintillation vials, two test tubes and glass vials total.
7. The 2 mL test tubes were placed into 15 mL BD Falcon Polystyrene Conical tubes, one for each. Tubes containing salt pellet solution filled reservoirs and Scintillation vial containing about 15 mL Milli Q water were placed in an incubator at 37°C and shaken at 100 rpm for 14 days, and the other samples were shaken for 3 days prior to the formaldehyde test.
8. 0.5 mL water was collected from each incubated sample and dispensed into a 2 mL test tube. One drop of formaldehyde test reagent FO-1 was added followed by mixing.
9. 0.5 mL of standard formaldehyde solutions, 10 mg/L, 20 mg/L and 40 mg/L, respectively, was dispensed into 2 mL test tubes, and one drop of reagent FO-1 was added into each tube followed by mixing.
10. The samples were tested using the test strips, and the color of the strips was compared. The corresponding result in mg/L HCHO was determined.

### RESULTS

### HCHO Test in ALZET^{®} Samples

| Sample | Strip color in test | Note |
|---|---|---|
| Salt Pellet Solution Filled Reservoirs | Slightly Brown | Strip color darker than the one from water and lighter than the one from HCHO 10 mg/L solution |
| Empty Reservoirs | Pink | No HCHO |
| Incubated Water in Glass Vials | Pink | No HCHO |
| Incubated Water in Plastic Test Tubes | Pink | No HCHO |
| Fresh Milli Q Water | Pink | No HCHO |
| 10 mg/L HCHO Solution | Brown | |
| 20 mg/L HCHO Solution | Dark Brown | |

### SUMMARY

In the HCHO permeation study, the salt pellet aqueous solution filled ALZET^{®} reservoir placed in water and incubated for 14 days at 37°C showed the presence of HCHO in the water samples since the color of strips from the samples was darker than the one from water. This result indicated that HCHO permeated into the water from the salt pellet solution through the reservoir. Since the color of strips of these samples was lighter than the one from standard HCHO 10 mg/L, the HCHO concentration of the samples was less than 10 mg/L.

### COMPARATIVE EXAMPLE 3

### OBJECTIVE

In Comparative Example 2, formaldehyde was found to permeate through the ALZET^{®} reservoir from the salt pellet solution. The present Comparative Example was to confirm the observation in Comparative Example 2. More concentrated salt pellet solution was used in the present Comparative Example, and the salt pellet solution filled reservoirs were incubated at 37°C for 14 days prior to the formaldehyde test.

### MATERIALS

**ALZET^{®} reservoirs: Model 200X**
**ALZET^{®} plugs**
**ALZET^{®} salt pellets, γ-irradiated (15 to 25 kGy) at Sterigenics International**
**Kraton^{™} copolymer in cyclohexane**
**ALZET^{®} 200X Filling Tubes**
**15 mL BD Falcon Polystyrene Conical tubes**
**2 mL plastic test tubes**
**Milli Q Water**
**20 ml Scintillation Glass Vials**
**Formaldehyde Test Kit, EM Quant**
**Formaldehyde standard solutions:**
   **HCHO concentrations at 10 mg/L, 20 mg/L, 40 mg/L, and 60 mg/L**

### EXPERIMENTAL

1. 4 salt pellets were placed in a 5 mL glass vial, and the salt pellets were weighed. 2 mL Milli Q water was added into the vial. The vial was placed on a Rugged Rotator to mix until all salt pellets were dissolved. The final salt pellet concentration was 0.583 g/mL.
2. A plug was secured in the mouth of the reservoir using cyclohexane glue and air dried at R.T. for 24 hours prior to use (6 reservoirs total).
3. About 0.2-0.25 mL of salt pellet solution was injected into each reservoir through the plug using a 1 mL syringe equipped with a filling tube.
4. The reservoirs were placed into 2 mL plastic test tubes, and Milli Q water was added into the test tubes to a level about 1-2 mm below the top of the reservoirs (about 0.5 mL).
5. The 2 mL test tubes were placed into 15 mL BD Falcon Polystyrene Conical tubes, one for each. The 15 mL BD tubes were placed in an incubator at 37°C and shaken at 100 rpm for 14 days prior to the formaldehyde test.
6. 0.5 mL water was collected from each incubated sample and dispensed into 2 mL test tube. One drop of formaldehyde test reagent FO-1 was added followed by mixing.
7. 0.5 mL of Milli Q water or standard formaldehyde solutions, 10 mg/L, 20 mg/L and 40 mg/L, respectively, was added into 2 mL test tubes, and one drop of reagent FO-1 was added into each tube followed by mixing.
8. The samples were tested using the test strips, and the color of the strips was compared. The corresponding result in mg/L HCHO was determined.

### RESULTS

### HCHO Test Results

| Sample | Strip color in test | Note |
|---|---|---|
| Salt Pellet Solution, Concentration: 0.583 g/mL | Dark Purple | Strip color same as or darker than the one from HCHO 40 mg/L solution |
| Water Outside the Salt Pellet Solution Filled Reservoirs | Brownish Pink | Strip color close to the one from HCHO 10 mg/L solution |
| Fresh Milli Q Water | Orange | |
| 10 mg/L HCHO Solution | Brownish Pink | |
| 20 mg/L HCHO Solution | Dark Brown | |
| 40 mg/L HCHO Solution | Purple | |

### SUMMARY

About 40 mg/L or greater of HCHO was found in the salt pellet solution with concentration of 0.538 g/mL. In the HCHO permeation study, the salt pellet aqueous solution with concentration of 0.538 g/mL filled ALZET^{®} reservoir placed in water and incubated for 14 days at 37°C showed the presence of HCHO in the water samples. The color of formaldehyde test strips from the permeation samples was the same as the one from formaldehyde 10 mg/L standard solution. This confirmed that HCHO permeated into the water from the salt pellet solution through the reservoir.

### COMPARATIVE EXAMPLE 4

### OBJECTIVE

The present Comparative Example involves determining if any formaldehyde in ALZET^{®} samples with or without γ-irradiation.

### MATERIALS

### 1) Non γ-irradiated ALZET^{®} samples:

**Salt Pellets (NaCl 45 wt%, PolyOx^{™} 40 wt% and PEG 8000 15 wt%)**
**PEG 8000**
**PolyOx^{™} WSR N-80 (Dow Chemical)**
**NaCl**

### 2) γ-Irradiated ALZET^{®} samples (dosage strength: 15.2-18.6 kGy at Sterigenics International)

**Salt Pellets (NaCl 45 wt%, PolyOx^{™} 40 wt% and PEG 8000 15 wt%)**
**PEG 8000**
**PolyOx^{™} WSR N-80 (Dow Chemical)**

**20 ml Scintillation Glass Vials**
**Milli Q Water**
**Formaldehyde Test Kit**

### EXPERIMENTAL

1. Salt pellets were placed into a 20 mL glass vial (3 salt pellets each), and 10 mL Milli Q water was added. The vials were placed on a Rugged Rotator to mix for 2 hours until the salt pellets were dissolved. The resulting solutions were a little cloudy.
2. Approximately 0.5 g of PEG, PolyOx^{™} or NaCl was placed into a 20 mL glass vial, and 10 mL Milli Q water was added followed by mixing on the Rugged Rotator until all dissolved.
3. 0.5 mL of each of the solutions was dispensed into 2 mL plastic test tubes, and one drop of formaldehyde test reagent FO-1 was added followed by shaking the tubes to mix.
4. 0.5 mL of standard formaldehyde solutions at 10, 20 and 40 mg/L, respectively, was dispensed into 2 mL test tubes, and one drop of FO-1 was added to each tube followed by mixing.
5. 0.5 mL Milli Q water was dispensed into a 2 mL test tube, and one drop of FO-1 was added followed by mixing.
6. The test samples were tested using the test strips, and the color of the strips was compared. The corresponding result in mg/L HCHO was determined.

### RESULTS

### HCHO Test Results in ALZET^{®} Samples

| Material | Irradiated? | Concentration (g/mL) | Color of Strip | Note |
|---|---|---|---|---|
| Salt Pellets | No | 0.581 | Orange | No HCHO; if yes, less than 10 mg/L |
| Salt Pellets | No | 0.080 | Orange | |
| Salt Pellets | Yes | 0.088 | Dark Brown | HCHO close to 20 mg/L |
| PEG | No | 0.503 | Orange | No HCHO; if yes, less than 10 mg/L |
| PEG | No | 0.500 | Orange | |
| PEG | Yes | 0.501 | Brownish Pink | HCHO close to 10 mg/L |
| PolyOx | No | 0.500 | Orange | No HCHO; if yes, less than 10 mg/mL |
| PolyOx | No | 0.501 | Orange | |
| PolyOx | Yes | 0.501 | Brownish Pink | HCHO close to 10 mg/L |
| PolyOx | Yes | 0.500 | Brownish Pink | |
| NaCl | No | 0.501 | Orange | No HCHO; if yes, less than 10 mg/L |
| Water | | | Orange | |
| Standard HCHO Solution 10 mg/L | | | Brownish Pink | |
| Standard HCHO Solution 20 mg/L | | | Dark Brown | |

The color of strips from the solutions of non γ-irradiated salt pellets, PEG, and PolyOx^{™} were the same as the one from Milli Q water. This indicated that no formaldehyde had been found in these test solutions, or if yes, the concentration of HCHO was less than 10 mg/L.

Regarding the formaldehyde test results on the solutions derived from γ-irradiated salt pellets, PEG and PolyOx^{™}, the color of the strip from the salt pellet solution was similar to the one from standard 20 mg/L HCHO solution. The color of strips from the irradiated PEG and two PolyOx^{™} solutions indicated HCHO at approximately 10 mg/L.

The color of the strip from the NaCl solution was the same as the one from water indicating no HCHO was present, or if yes, it must be less than 10 mg/L.

### SUMMARY

Either no formaldehyde was found in the solutions of non γ-irradiated salt pellet, PEG, PolyOx^{™} and NaCl, or if present, the HCHO concentration was less than 10 mg/L. In comparison with the non γ-irradiated samples, solutions derived from the γ-irradiated salt salt pellets, PEG, and PolyOx^{™} signaled the presence of HCHO. When the salt pellet concentration was 0.088 g/mL, the formaldehyde was approximately 20 mg/L. In the solution containing 0.5 g/mL of γ-irradiated PEG or PolyOx^{™}, the color of strip showed that the HCHO concentration was approximately 10 mg/L.

The γ-irradiation with dosing strength of 15.2 kGy to 18.6 kGy was found to cause the formaldehyde formation in the salt pellets, PEG and PolyOx^{™}.

### EXAMPLE 1

### OBJECTIVES

In Comparative Example 4, it was found that formaldehyde was generated in the salt pellet samples after γ-irradiation. In an effort to prevent the formation of formaldehyde, BHT (butylated hydroxytoluene) was added into the salt pellet formation as an antioxidant. In addition, Methocel^{™} was used to replace the PolyOx^{™} in the salt pellet formation. Also, HEC (hydroxyethyl cellulose) was used as a substitute for PEG. All salt pellet formulations were hot-melt processed in an Extrusion Plastometer. After extrusion the formulation samples underwent γ-irradiation. Formaldehyde testing was performed on salt pellet-derived aqueous solutions (before or after γ-irradiation).

### MATERIALS AND INSTRUMENTS

**BHT (butylated hydroxytoluene)**
**PolyOx^{™} WSR N-80, Dow Chemical**
**Methocel^{™} E5 Premium LV (hydroxypropyl methylcellulose), Dow Chem.**
**HEC (hydroxyethyl cellulose), WF**
**PEG 8000 (polyethylene glycol)**
**Sodium Chloride**
**Formaldehyde Standard Solutions:**
   **HCHO concentrations were 10 mg/L and 20 mg/L**
**Milli Q Water**
**20 ml Scintillation Glass Vials**
**Formaldehyde Test Kit, EM Quant**
**2 mL plastic test tubes**
**Extrusion Plastometer, Tinius Olsen, Model: MP600 Controller/Timer**

### EXPERIMENTAL

1. The salt pellets were comprised of NaCl 45 wt%, PolyOx^{™} 40 wt%, and PEG 8000 15 wt%. All salt pellet formulations were maintained the same whenever PolyOx^{™} or PEG was substituted with Methocel^{™} or HEC.
2. BHT was added to the salt pellet formulations at three different concentrations, 1 wt%, 3 wt%, and 5 wt%, respectively. BHT was ground using a mill jar into a fine powder and then sieved through a size 30 mesh prior to use. One group without BHT was prepared as a control.
3. The powder containing each excipient was placed in a 20 mL Scintillation vial and mixed on a Rugged Rotator overnight to uniformity.
4. The mixture including the powder was loaded in the Extrusion Plastometer and hot-melt extruded at the below-listed temperatures. The extruded material was placed in a 20 mL Scintillation vial.
5. The irradiated samples were γ-irradiated at 27.5 to 32.5 kGy at Sterigenics International.
6. About 0.5 g of non-irradiated or γ-irradiated salt pellet material was placed in a 20 mL glass vial, and 10 mL of Milli Q water was added. The vials were placed on a Rugged Rotator and mixed until the solution was saturated. Most of the samples were not completely dissolved.
7. Solution samples were centrifuged at 1200 rpm for 10 min, and 0.5 mL of the upper layer clear solution after centrifugation was dispensed into a 2 mL plastic test tube. One drop of formaldehyde test reagent FO-1 was added followed by mixing.
8. 0.5 mL of Milli Q water or standard formaldehyde solutions, 10 mg/L and 20 mg/L, respectively, was dispensed into 2 mL test tubes, and one drop of reagent FO-1 was added into each tube followed by mixing.
9. The samples were tested using the test strips, and the color of the strips was compared. The corresponding result in mg/L HCHO was determined.

### RESULTS AND DISCUSSION

### Addition of BHT to ALZET^{®} Salt Pellet and Modified Salt Pellet Formulations and Applied Hot-Melt Extrusion Process

| Salt Pellet Formulation | Composition (wt%) | BHT (wt%) | Temperature for Extrusion, °C | Color of Material after Extrusion |
|---|---|---|---|---|
| 1 | NaCl/PolyOx/PEG 45 : 40 : 15 | 1 | 150 | Off-White |
| 2 | | 3 | | |
| 3 | | 5 | | |
| 4 | | 0 | | |
| 5 | NaCl/PolyOx/HEC 45 : 40 : 15 | 1 | 170 | Off-White to Light Brown |
| 6 | | 3 | | |
| 7 | | 5 | | |
| 8 | | 0 | | |
| 9 | NaCl/Methocel/PEG 45 : 40 : 15 | 1 | 230 | Light Brown |
| 10 | | 3 | | |
| 11 | | 5 | | |
| 12 | | 0 | | |
| 13 | NaCl/Methocel/HEC 45 : 40 : 15 | 1 | 250 | Brown |
| 14 | | 3 | | |
| 15 | | 5 | | |
| 16 | | 0 | | |

### Preparation of Non-Irradiated Salt Pellet Solutions in H₂O for Formaldehyde Test

| Salt Pellet Formulation | Mixing time (h) | Pellet Conc. (g/mL) | Dissolved completely? | Color of Solution |
|---|---|---|---|---|
| 1 | 22 | 0.088 | No | Colorless |
| 2 | | 0.088 | No | Colorless |
| 3 | | 0.089 | No | Colorless |
| 4 | | 0.088 | Almost, only few particles in solution | Colorless |
| 5 | 22 | 0.088 | No | Colorless |
| 6 | | 0.088 | No | Colorless |
| 7 | | 0.088 | No | Colorless |
| 8 | | 0.088 | Almost, gelly material in solution | Colorless |
| 9 | 22 | 0.088 | No | Light Brown |
| 10 | | 0.088 | No | Light Brown |
| 11 | | 0.088 | No | Light Green |
| 12 | | 0.088 | Almost, only few particles in solution | Brown |
| 13 | 22 | 0.088 | No | Brown |
| 14 | | 0.088 | No | Brown |
| 15 | | 0.088 | No | Brown |
| 16 | | 0.088 | Almost, some particles in solution | Dark Brown |

### Preparation of γ-Irradiated Salt Pellet Solutions in H₂O for Formaldehyde Test

| Salt Pellet Formulation | Mixing time (h) | Pellet Conc. (g/mL) | Dissolved completely? | Color of Solution |
|---|---|---|---|---|
| 1 | 64 | 0.056 | Almost | Light Green |
| 2 | | 0.055 | No | Light Green |
| 3 | | 0.052 | No | Light Green |
| 4 | | 0.088 | Almost, only few particles in solution | Almost Colorless |
| 5 | 64 | 0.045 | No | Light Green |
| 6 | | 0.046 | No | Light Green |
| 7 | | 0.04 | No | Off-White |
| 8 | | 0.067 | Almost, Cloudy | Colorless |
| 9 | 64 | 0.057 | No | Light Green |
| 10 | | 0.05 | No | Light Brown |
| 11 | | 0.045 | No | Light Green |
| 12 | | 0.088 | Yes | Colorless |
| 13 | 64 | 0.058 | No | Light Brown |
| 14 | | 0.056 | No | Light Brown |
| 15 | | 0.056 | No | Off-White |
| 16 | | 0.07 | No | Brown |

In the hot-melt extrusion process, temperatures applied to the Salt Pellet Formulations 1-4 and 5-8 were 150°C and 170°C, respectively. The color of the extruded materials was white, and their solutions in H₂O were colorless, which indicated that the temperatures were in the range the excipients in the salt pellets could tolerate. In order to melt and extrude Salt Pellet Formulations 9-12 and 13-16, a temperature in the range of 230°C - 250°C was needed. Under this condition, the color of material after extrusion became brown or even darker. Their solutions were brown.

### HCHO Test Results

### Non-Irradiated Salt Pellet Solutions

### Formaldehyde Test on None γ -Irradiated Salt Pellet Solutions

| Salt Pellet Formulation | Composition (%) | BHT (%) | Color of Strip | Note |
|---|---|---|---|---|
| 1 | NaCl/PolyOx/PEG 45 : 40 : 15 | 1 | Orange | No HCHO |
| 2 | | 3 | Orange | |
| 3 | | 5 | Orange | |
| 4 | | 0 | Orange | |
| 5 | NaCl/PolyOx/HEC 45 : 40 : 15 | 1 | Orange | |
| 6 | | 3 | Orange | |
| 7 | | 5 | Orange | |
| 8 | | 0 | Orange | |
| 9 | NaCl/Methocel/PEG 45 : 40 : 15 | 1 | Orange | |
| 10 | | 3 | Orange | |
| 11 | | 5 | Orange | |
| 12 | | 0 | Very Light brownish Pink | To be confirmed |
| 13 | NaCl/Methocel/HEC 45 : 40 : 15 | 1 | Orange | No HCHO |
| 14 | | 3 | Orange | |
| 15 | | 5 | Orange | |
| 16 | | 0 | Orange | |
| Water | H2O | NA | Orange | |
| HCHO Standard Solution, 10 mg/L | HCHO, 10 mg/L | NA | Brownish Pink | |

In the formaldehyde test on the non-irradiated salt pellet solutions, the color of strips from all the test solutions was the same as it from H₂O, except the solution from Salt Pellet Formulation 12 gave a test strip color that was slightly brownish pink. This observation indicated that no HCHO was present in all the test solutions made from non-irradiated salt pellets regardless of whether the salt pellet formulation contained BHT or not.

### γ-Irradiated Salt Pellet Solutions

### Formaldehyde Test on γ-Irradiated Salt Pellet Solutions

| Salt Pellet Formulation | Composition (wt%) | BHT (wt%) | Color of Strip | Note |
|---|---|---|---|---|
| 1 | NaCl/PolyOx/PEG 45 : 40 : 15 | 1 | Orange | No HCHO |
| 2 | | 3 | Orange | |
| 3 | | 5 | Orange | |
| 4 | | 0 | Brownish Pink | HCHO 10 mg/L |
| 5 | NaCl/PolyOx/HEC 45 : 40 : 15 | 1 | Orange | No HCHO |
| 6 | | 3 | Orange | |
| 7 | | 5 | Orange | |
| 8 | | 0 | Brownish Pink | HCHO 10 mg/L |
| 9 | NaCl/Methocel/PEG 45 : 40 : 15 | 1 | Brownish Pink | HCHO 10 mg/L |
| 10 | | 3 | Orange | No HCHO |
| 11 | | 5 | Orange | |
| 12 | | 0 | Dark Brown | HCHO 20 mg/L |
| 13 | NaCl/Methocel/HEC 45 : 40 : 15 | 1 | Brownish Pink | HCHO 10 mg/L |
| 14 | | 3 | Brownish Pink | |
| 15 | | 5 | Brownish Pink | |
| 16 | | 0 | Dark Brown | HCHO 20 mg/L |
| Water | H₂O | NA | Orange | |
| HCHO Standard Solution, 10 mg/L | HCHO, 10 mg/L | NA | Brownish Pink | |
| HCHO Standard Solution, 20 mg/L | HCHO, 20 mg/L | NA | Dark Brown | |

One hypothesis of this study was that BHT may prevent the formation of formaldehyde, which is likely a result of the oxidation of PolyOx^{™} or/and PEG in the γ-irradiation process, and the anti-oxidation will be dependent on the concentration of BHT in the salt pellet formulation. In the test of solutions prepared from γ-irradiated salt pellet, all four solutions without BHT gave brownish pink or dark brown color to the test strips. This observation indicated that γ-irradiation caused the formation of formaldehyde in the salt pellets without BHT in the formulations. For Salt Pellet Formulations 1-3 and 5-7 containing BHT ranging from 1 wt% to 5 wt%, the orange color of the test strips was the same as the one from H₂O, showing no HCHO in the solutions and suggesting that the BHT blocked the oxidation of excipients to generate the formaldehyde. For Salt Pellet Formulation 9 containing 1 wt% BHT and Salt Pellet Formulations 13-15 containing BHT at 1 wt%, 3 wt% or 5 wt%, respectively, the brownish pink color of the test strips indicated that HCHO was formed in the samples at a concentration of approximately 10 mg/L. No HCHO was found in the samples of Salt Pellet Formulations 10 and 11 containing 3 wt% or 5 wt%, respectively, of BHT suggesting the anti-oxidation effectiveness depended on the concentration of BHT in the salt pellet.

Substitution of PolyOx^{™} or PEG with Methocel^{™} or HEC in the salt pellet formulation showed negative impact on the prevention of formaldehyde formation.

### SUMMARY

In order to prevent formaldehyde formation in the salt pellet after γ-irradiation, BHT was used as an antioxidant in the salt pellets. In particular, BHT was added to the salt pellet formulations at 1 wt%, 3 wt%, or 5 wt%. It was found that BHT may prevent the formation of formaldehyde in salt pellet formulation NaCl / PolyOx^{™} / PEG 45:40:15 or revised formulation NaCl / PolyOx^{™} / HEC 45:40:15. The BHT was effective at a concentration as low as 1 wt%. Results showed the anti-oxidation effectiveness of BHT was dependent on its concentration in the salt pellet. Substitution of PolyOx^{™} or PEG with Methocel^{™} or HEC in the salt pellet formulation increased formaldehyde formation. Salt Pellet Formulations 5-16 required higher temperature in the hot-melt extrusion process than Salt Pellet Formulations 1-4.

### EXAMPLE 2

### BACKGROUND AND OBJECTIVES

In Example 1, it was found that BHT can prevent the formation of formaldehyde in γ-irradiated salt pellet formulations comprising NaCl / PolyOx^{™} / PEG at a weight ratio of 45:40:15. In Example 1, 1 wt%, 3 wt%, or 5 wt% of BHT was added to the salt pellet formulation. The BHT was effective at a concentration as low as 1 wt%. Results showed that the anti-oxidation effectiveness of BHT was dependent on its concentration in the salt pellet, which was γ-irradiated at a dose of 27.5 kGy to 32.5 kGy.

The present Example is directed to confirming the effect of the BHT on the prevention of formaldehyde formation in the salt pellets during γ-irradiation. Salt pellets underwent γ-irradiation at a dose of 15.0 kGy to 25.0 kGy. Two different lots of each of the PolyOx^{™} and PEG were used.

The study method was similar to that of Example 1. Different salt pellet formulations underwent hot-melt process in an Extrusion Plastometer after mixing. The formulation samples after extrusion underwent γ-irradiation. Formaldehyde tests were performed on the salt pellet-derived aqueous solutions (before or after γ-irradiation).

### MATERIALS AND INSTRUMENTS

**BHT (butylated hydroxytoluene)**
**PolyOx^{™} WSR N-80, Dow Chemical, Lot PO-A**
**PolyOx^{™} WSR N-80, Dow Chemical, Lot PO-B**
**PEG 8000 (polyethylene glycol), Lot PEG-A**
**PEG 8000 (polyethylene glycol), Lot PEG-B**
**Sodium Chloride**
**Formaldehyde Standard Solutions:**
   **HCHO concentrations were 10 mg/L and 20 mg/L**
**Milli Q Water**
**20 ml Scintillation Glass Vials**
**Formaldehyde Test Kit, EM Quant**
**2 mL plastic test tubes**
**Extrusion Plastometer, Tinius Olsen, Model: MP600 Controller/Timer**

### EXPERIMENTAL

1. The salt pellets were comprised of NaCl 45 wt%, PolyOx^{™} 40 wt%, and PEG 15 wt%.
2. BHT was added to the salt pellet formulations at five different concentrations: 1.0 wt%, 1.5 wt%, 2.0 wt%, 2.5 wt%, 3.0 wt%, and 5.0 wt%. BHT was ground using a mill jar into a fine powder and then sieved through a size 30 mesh prior to use. One group without BHT was prepared as a control.
3. The powder containing each excipient was placed in a 20 mL Scintillation vial and mixed on a Rugged Rotator overnight to uniformity.
4. The mixture was loaded in the Extrusion Plastometer and hot-melted and extruded at 158°C. The extruded material was placed in a 20 mL Scintillation vial.
5. The irradiated samples were γ-irradiated at a dose of 15.0 kGy to 25.0 kGy at Sterigenics International.
6. About 0.5 g of the non-irradiated or γ-irradiated salt pellet material was placed in a 20 mL glass vial, and 10 mL of Milli Q water was added. The vials were placed on a Rugged Rotator to mix until the solutions were saturated. Most of the samples were not completely dissolved.
7. Solution samples were centrifuged at 10,000 rpm for 10 min, and 0.5 mL of the upper layer clear solution after centrifugation was dispensed into a 2 mL plastic test tube. One drop of formaldehyde test reagent FO-1 was added followed by mixing.
8. 0.5 mL of Milli Q water or standard formaldehyde solutions, 10 mg/L and 20 mg/L, respectively, was dispensed into 2 mL test tubes, and one drop of reagent FO-1 was added into each tube followed by mixing.
9. The samples were tested using the test strips, and the color of the strips was compared. The corresponding result in mg/L HCHO was determined.

### RESULTS AND DISCUSSION:

### Preparation of Non-Irradiated Salt Pellet Solutions in H₂O for Formaldehyde Test (1)

| Salt Pellet Formulation | BHT (wt%) | PolyOx Lot | PEG Lot | Pellet Conc. (g/mL) | Dissolved completely? | Color of Solution |
|---|---|---|---|---|---|---|
| 17 | 1.0 | PO-A | PEG-A | 0.055 | No | Colorless |
| 18 | 1.5 | | | 0.053 | No | Colorless |
| 19 | 2.0 | | | 0.052 | No | Colorless |
| 20 | 2.5 | | | 0.053 | No | Colorless |
| 21 | 3.0 | | | 0.052 | No | Colorless |
| 22 | 4.0 | | | 0.050 | No | Colorless |
| 23 | 0 | | | 0.086 | Almost | Colorless |
| 24 | 1.0 | PO-B | PEG-A | 0.056 | No | Colorless |
| 25 | 1.5 | | | 0.052 | No | Colorless |
| 26 | 2.0 | | | 0.053 | No | Colorless |
| 27 | 2.5 | | | 0.052 | No | Colorless |
| 28 | 3.0 | | | 0.052 | No | Colorless |
| 29 | 4.0 | | | 0.050 | No | Colorless |
| 30 | 0 | | | 0.090 | No | Colorless |

### Preparation of Non-Irradiated Salt Pellet Solutions in H₂O for Formaldehyde Test (2)

| Salt Pellet Formulation | BHT (wt%) | PolyOx Lot | PEG Lot | Pellet Conc. (g/mL) | Dissolved completely? | Color of Solution |
|---|---|---|---|---|---|---|
| 31 | 1.0 | PO-A | PEG-B | 0.058 | No | Colorless |
| 32 | 1.5 | | | 0.057 | No | Colorless |
| 33 | 2.0 | | | 0.053 | No | Colorless |
| 34 | 2.5 | | | 0.055 | No | Colorless |
| 35 | 3.0 | | | 0.055 | No | Colorless |
| 36 | 4.0 | | | 0.055 | No | Colorless |
| 37 | 0 | | | 0.093 | Almost | Colorless |
| 38 | 1.0 | PO-B | PEG-B | 0.056 | No | Colorless |
| 39 | 1.5 | | | 0.058 | No | Colorless |
| 40 | 2.0 | | | 0.057 | No | Colorless |
| 41 | 2.5 | | | 0.058 | No | Colorless |
| 42 | 3.0 | | | 0.058 | No | Colorless |
| 43 | 4.0 | | | 0.059 | No | Colorless |
| 44 | 0 | | | 0.094 | Almost | Colorless |

### Preparation of γ-Irradiated Salt Pellet Solutions in H₂O for Formaldehyde Test (1)

| Salt Pellet Formulation | BHT (wt%) | PolyOx Lot | PEG Lot | Pellet Conc. (g/mL) | Dissolved completely? | Color of Solution |
|---|---|---|---|---|---|---|
| 17 | 1.0 | PO-A | PEG-A | 0.052 | No | Light Green |
| 18 | 1.5 | | | 0.053 | No | Light Green |
| 19 | 2.0 | | | 0.056 | No | Light Green |
| 20 | 2.5 | | | 0.057 | No | Light Green |
| 21 | 3.0 | | | 0.052 | No | Light Green |
| 22 | 4.0 | | | 0.053 | No | Light Green |
| 23 | 0 | | | 0.090 | Almost | Near Colorless |
| 24 | 1.0 | PO-B | PEG-A | 0.053 | No | Near Colorless |
| 25 | 1.5 | | | 0.053 | No | Near Colorless |
| 26 | 2.0 | | | 0.053 | No | Light Green |
| 27 | 2.5 | | | 0.052 | No | Light Green |
| 28 | 3.0 | | | 0.052 | No | Near Colorless |
| 29 | 4.0 | | | 0.052 | No | Near Colorless |
| 30 | 0 | | | 0.093 | Almost | Colorless |

### Preparation of γ-Irradiated Salt Pellet Solutions in H₂O for Formaldehyde Test (2)

| Salt Pellet Formulation | % BHT | PolyOx Lot | PEG Lot | Pellet Conc. (g/mL) | Dissolved completely? | Color of Solution |
|---|---|---|---|---|---|---|
| 17 | 1.0 | PO-A | PEG-A | 0.053 | No | Light Green |
| 18 | 1.5 | | | 0.054 | No | Light Green |
| 19 | 2.0 | | | 0.063 | No | Light Green |
| 20 | 2.5 | | | 0.060 | No | Light Green |
| 21 | 3.0 | | | 0.056 | No | Light Green |
| 22 | 4.0 | | | 0.055 | No | Light Green |
| 23 | 0 | | | 0.094 | Almost | Near Colorless |
| 24 | 1.0 | PO-B | PEG-A | 0.056 | No | Near Colorless |
| 25 | 1.5 | | | 0.054 | No | Near Colorless |
| 26 | 2.0 | | | 0.064 | No | Light Green |
| 27 | 2.5 | | | 0.064 | No | Light Green |
| 28 | 3.0 | | | 0.055 | No | Near Colorless |
| 29 | 4.0 | | | 0.055 | No | Near Colorless |
| 30 | 0 | | | 0.094 | Almost | Colorless |

In the hot-melt extrusion process, the temperature applied to all samples was 158°C. The color of the extruded materials was white, and their solutions in H₂O were colorless without γ-irradiation. The γ-irradiated salt pellets were light yellow, and their solutions were light green indicating that BHT-quinone, the oxidation product of BHT, was formed during the γ-irradiation (H. Daun, S.G. Gilbert and J. Giacin, Journal of the American Oil Chemists' Society, V 51, Number 9, 404-406). This was consistent with the observation in Example 1.

### HCHO Test Results

### Non-Irradiated Salt Pellet Solutions

### Formaldehyde Test on Non-Irradiated Salt Pellet Solutions (1)

| Salt Pellet Formulation | BHT (wt%) | PolyOx Lot | PEG Lot | Color of Test Strip | Note |
|---|---|---|---|---|---|
| 17 | 1.0 | PO-A | PEG-A | Orange | No HCHO |
| 18 | 1.5 | | | Orange | |
| 19 | 2.0 | | | Orange | |
| 20 | 2.5 | | | Orange | |
| 21 | 3.0 | | | Orange | |
| 22 | 4.0 | | | Orange | |
| 23 | 0 | | | Orange with very light Pink | HCHO less than 10 mg/L |
| 24 | 1.0 | PO-B | PEG-A | Orange | No HCHO |
| 25 | 1.5 | | | Orange | |
| 26 | 2.0 | | | Orange | |
| 27 | 2.5 | | | Orange | |
| 28 | 3.0 | | | Orange | |
| 29 | 4.0 | | | Orange | |
| 30 | 0 | | | Very Light Brownish Pink | HCHO less than 10 mg/L |
| Water | H2O | | | Orange | |
| HCHO Standard Solution, 10 mg/L | HCHO, 10 mg/L | | | Brownish Pink | |

### Formaldehyde Test on Non-Irradiated Salt Pellet Solutions (2)

| Salt Pellet Formulation | BHT (wt%) | PolyOx Lot | PEG Lot | Color of Test Strip | Note |
|---|---|---|---|---|---|
| 31 | 1.0 | PO-A | PEG-B | Orange | No HCHO |
| 32 | 1.5 | | | Orange | |
| 33 | 2.0 | | | Orange | |
| 34 | 2.5 | | | Orange | |
| 35 | 3.0 | | | Orange | |
| 36 | 4.0 | | | Orange | |
| 37 | 0 | | | Brownish Pink | HCHO 10 mg/L |
| 38 | 1.0 | PO-A | PEG-B | Orange | No HCHO |
| 39 | 1.5 | | | Orange | |
| 40 | 2.0 | | | Orange | |
| 41 | 2.5 | | | Orange | |
| 42 | 3.0 | | | Orange | |
| 43 | 4.0 | | | Orange | |
| 44 | 0 | | | Brownish Pink | HCHO 10 mg/L |
| Water | H2O | | | Orange | |
| HCHO Standard Solution, 10 mg/L | HCHO, 10 mg/L | | | Brownish Pink | |
| HCHO Standard Solution, 20 mg/L | HCHO, 20 mg/L | | | Dark Brown | |

As shown in the two Tables immediately above, in the formaldehyde test on the non-irradiated salt pellet solutions with Lot PO-A or PO-B in combination with Lot PEG-A or PEG-B, which contained BHT from 1 wt% to 4 wt%, the color of strips from all the test solutions was the same as it was from H₂O. This observation indicated that no HCHO was present in these test solutions prepared from non-irradiated salt pellet containing 1 wt% to 4 wt% of BHT. Solutions prepared from salt pellets lacking BHT gave the test strip a slightly brownish pink color showing the presence of HCHO. This observation indicated that hot-melt extrusion at 158°C may cause the oxidation of excipient(s) to generate HCHO. In Example 1, no HCHO was found in the solution prepared from non-irradiated salt pellet with or without BHT in the formulations after extrusion at 150°C.

### γ-Irradiated Salt Pellet Solutions

### Formaldehyde Test on γ-Irradiated Salt Pellet Solutions (1)

| Salt Pellet Formulation | % BHT | PolyOx Lot | PEG Lot | Color of Test Strip | Note |
|---|---|---|---|---|---|
| 17 | 1.0 | PO-A | PEG-A | Orange | No HCHO |
| 18 | 1.5 | | | Orange | |
| 19 | 2.0 | | | Orange | |
| 20 | 2.5 | | | Orange | |
| 21 | 3.0 | | | Orange | |
| 22 | 4.0 | | | Orange | |
| 23 | 0 | | | Dark Brown | HCHO about 10-20 mg/L |
| 24 | 1.0 | PO-B | PEG-A | Orange | No HCHO |
| 25 | 1.5 | | | Orange | |
| 26 | 2.0 | | | Orange | |
| 27 | 2.5 | | | Orange | |
| 28 | 3.0 | | | Orange | |
| 29 | 4.0 | | | Orange | |
| 30 | 0 | | | Dark Brown | HCHO about 10-20 mg/L |
| Water | H2O | | | Orange | |
| HCHO Standard Solution, 10 mg/L | HCHO, 10 mg/L | | | Brownish Pink | |
| HCHO Standard Solution, 20 mg/L | HCHO, 20 mg/L | | | Dark Brown | |

### Formaldehyde Test on γ-Irradiated Salt Pellet Solutions (2)

| Salt Pellet Formulation | % BHT | PolyOx Lot# | PEG Lot# | Color of Test Strip | Note |
|---|---|---|---|---|---|
| 31 | 1.0 | PO-A | PEG-B | Orange | No HCHO |
| 32 | 1.5 | | | Orange | |
| 33 | 2.0 | | | Orange | |
| 34 | 2.5 | | | Orange | |
| 35 | 3.0 | | | Orange | |
| 36 | 4.0 | | | Orange | |
| 37 | 0 | | | Brownish Pink | HCHO about 10 mg/L |
| 38 | 1.0 | PO-B | PEG-B | Orange | No HCHO |
| 39 | 1.5 | | | Orange | |
| 40 | 2.0 | | | Orange | |
| 41 | 2.5 | | | Orange | |
| 42 | 3.0 | | | Orange | |
| 43 | 4.0 | | | Orange | |
| 44 | 0 | | | Brownish Pink | HCHO about 10 mg/L |
| Water | H2O | | | Orange | |
| HCHO Standard Solution, 10 mg/L | HCHO, 10 mg/L | | | Brownish Pink | |
| HCHO Standard Solution, 20 mg/L | HCHO, 20 mg/L | | | Dark Brown | |

In Example 1, BHT was shown to prevent oxidation of the PolyOx^{™} or PEG to form formaldehyde. As shown in the two Tables immediately above, in the solutions prepared from γ-irradiated salt pellets, all four solutions without BHT gave a brownish pink to dark brown color to the test strips, which suggested that the formaldehyde concentration ranged from 10 mg/L to 20 mg/L. This observation indicated that γ-irradiation caused the formation of formaldehyde in the salt pellets without BHT. In all the solutions containing BHT at 1 wt% up to 4 wt%, the orange color of test strips, which was the same as the one from H₂O, showed no HCHO in the solutions suggesting that the BHT blocked the oxidation of excipients. Since the color of the strips from these solutions was the same, BHT at 1 wt% was enough to block the oxidation of the excipient(s) and prevent the formation of formaldehyde. In present Example 2, the dose used in the γ-irradiation ranged from 15.0 kGy to 25.0 kGy. In Example 1, the dose of γ-irradiation ranged from 27.5 kGy to 32.5 kGy, and the results showed that the anti-oxidation effectiveness depended on the concentration of BHT in the salt pellets.

No difference was found using two different lots of PolyOx^{™} or PEG in the salt pellets in the study.

### SUMMARY

In order to prevent formaldehyde formation in the γ-irradiated salt pellets, BHT was used as an antioxidant in the salt pellets. The present Example confirmed the effect of BHT on the oxidation of excipient(s) in the salt pellets. In salt pellet formulations containing BHT at 1 wt%, 1.5 wt%, 2 wt%, 2.5 wt%, 3 wt%, or 4 wt%, the formation of formaldehyde was prevented in formulations based on NaCl / PolyOx^{™} / PEG at a weight ratio of 45:40:15. The BHT showed effect at a concentration as low as 1 wt%. This observation was consistent with the results in Example 1.

Unlike the observations in Example 1, the present Example showed that the anti-oxidation effectiveness of BHT was not dependent on its concentration in the salt pellets due to the lower dose of γ-irradiation used in the irradiation (15.0 kGy to 25.0 kGy). Thus, the dose of 15.0 kGy to 25.0 kGy is preferred.

### EXAMPLE 3

### OBJECTIVE

The present Example involves determining if any formaldehyde was present in γ-irradiated salt sleeves (solute layers).

### MATERIALS

**γ-irradiated ALZET^{®} salt sleeve samples (dose: 15** - **25 kGy at Sterigenics International):**
   **2 mL salt sleeves sized to fit 2 MLX ALZET^{®} models**
   **200 µl salt sleeves sized to fit 200X ALZET^{®} models**
   **100 µl salt sleeves sized to fit 100X ALZET^{®} models**
   **All of these salt sleeves contained 1.5 wt% BHT in NaCl / PolyOx^{™} / PEG at a weight ratio of 45:40:15**
**20 ml Scintillation Glass Vials**
**Purified Water USP**
**Formaldehyde Test Kit, EM Quant**

### EXPERIMENT

1. Salt sleeves were placed into a 20 mL glass vial and weighed. Purified water was added into each vial to have a salt sleeve concentration of about 0.053 g/mL (two vials for each model salt sleeve). One vial containing purified water was prepared as a control. The vials were placed on a Rugged Rotator to mix overnight. Materials were dissolved into the water almost completely.
2. 0.5 mL of each of the solutions was dispensed into respective 2 mL plastic test tubes, and one drop of formaldehyde test reagent FO-1 was added. The tubes were shaken to mix the contents.
3. 0.5 mL of standard formaldehyde solutions, 10 and 20 mg/L, was dispensed into 2 mL test tubes, and one drop of FO-1 was added to each tube followed by mixing.
4. 0.5 mL of the purified water control sample was dispensed into a 2 mL test tube, and one drop of FO-1 was added followed by mixing.
5. The samples were tested using the test strips, and the color of the strips was compared. The corresponding result in mg/L HCHO was determined.

### RESULTS

### HCHO Test Results in γ-Irradiated ALZET^{®} Salt Sleeve Solutions

| Material | Size/Model | Color of Strip | Note |
|---|---|---|---|
| Salt Sleeves | 2 MLX | Orange | No HCHO; if yes, less than 10 mg/L |
| Salt Sleeves | 2 MLX | Orange | |
| Salt Sleeves | 200X | Orange | |
| Salt Sleeves | 200X | Orange | |
| Salt Sleeves | 100X | Orange | |
| Salt Sleeves | 100X | Orange | |
| Purified Water | | Orange | |
| Standard HCHO Solution 10 mg/L | | Brownish Pink | |
| Standard HCHO Solution 20 mg/L | | Dark Brown | |

The color strips from all six salt sleeve solutions was the same as the one from purified water. This indicated that no formaldehyde was found in these four test solutions, or if yes, the concentration of HCHO was less than 10mg/L.

## Claims

1. A dispenser comprising:
a flexible container having a closed end and an opposed open end;
an osmotically-effective solute composition encapsulating at least a portion of the container, the solute composition comprising
- antioxidant in an amount ranging from 0.5 wt% to 20 wt%, based on weight of the solute composition, wherein the antioxidant comprises at least one member selected from butylated hydroxytoluene (BHT), butylated hydroxyanisole (BHA), thioethers, histidine, cysteine, tryptophan, tyrosine, ascorbic acid, vitamin E, ascorbyl palmitate, erythorbic acid, potassium metabisulfite, propyl gallate, sodium ascorbate, sodium bisulfate, sodium metabisulfite, sodium sulfite, sodium formaldehyde sulfoxylate, and thymol,
- a polymer comprising at least one of polyethylene oxide and polyethylene glycol, and
- a salt;
a semipermeable membrane encapsulating the osmotically-effective solute composition;
a cap having a sealing surface adapted for sealing engagement with the open end of the container; and
a port extending from an interior of the container and through the cap.

2. The dispenser of claim 1, wherein the antioxidant comprises BHT.

3. The dispenser of claim 1 or 2, wherein the flexible container is formed as a bag adapted to contain a fluid composition.

4. The dispenser of claim 3, wherein the dispenser comprises a flow moderator that comprises a rigid cylindrical tube and the cap, wherein the tube and the cap are made from plastics or metals.

5. The dispenser of any one of claims 1 to 4, wherein the semipermeable membrane comprises at least one polymer that is selected from:
(i) cellulose acetate butyrate, cellulose acetate propionate, polymethylmethacrylate, and ethyl cellulose; or
(ii) cellulose acetate butyrate and mixtures of cellulose acetate butyrate and ethyl cellulose; or
(iii) cellulose acetate propionate and mixtures of cellulose acetate propionate and ethyl cellulose; or
(iv) polymethylmethacrylate and mixtures of polymethylmethacrylate and ethyl cellulose.

6. The dispenser of any one of claims 1 to 5, wherein the semipermeable membrane further comprises at least one solvent, wherein the at least one solvent is selected from:
(i) acetone, methylene chloride, water, and lower alkanol having 1-8 carbon atoms; or
(ii) acetone, a mixture of acetone and water, a mixture of acetone and methanol, and a mixture of acetone and ethanol.

7. The dispenser of any one of claims 1 to 6, wherein the container has an arcuate, outer edge formed at its open end adjacent its junction with the sealing surface of the cap.

8. The dispenser of claim 7, wherein the arcuate edge has a radius of curvature between 0.254 mm (0.01 inches) and 2.29 mm (0.09 inches) or between 0.254 mm (0.01 inches) and 2.03 mm (0.08 inches) or between 0.245 mm (0.01 inches) and 1.78 mm (0.07 inches).

9. The dispenser of any one of claims 1 to 8, further comprising an active agent in the flexible container.

10. A dispenser as defined in any one of claims 1 to 9 for use in delivery of the active ingredient to a subject.

11. The dispenser of any one of claims 1 to 10, wherein the salt comprises NaCl.

12. The dispenser of any one of claims 1 to 11, wherein the salt is present in an amount ranging from 20 wt% to 60 wt% based on weight of the solute composition.

13. The dispenser of any one of claims 1 to 12, wherein the polymer comprising at least one of polyethylene oxide and polyethylene glycol is a polymer that comprises polyethylene oxide, wherein the polyethylene oxide is optionally present in an amount ranging from 20 wt% to 55 wt% based on weight of the solute composition.

14. The dispenser of any one of claims 1 to 13, wherein the polymer comprising at least one of polyethylene oxide and polyethylene glycol is a polymer that comprises polyethylene glycol, wherein the polyethylene glycol is optionally present in an amount ranging from 5 wt% to 25 wt% based on weight of the solute composition.

15. The dispenser of any one of claims 1 to 14, wherein
(i) the antioxidant comprises BHT; or
(ii) the antioxidant is present in an amount ranging from 0.5 wt% to 10 wt% or from 1 wt% to 5 wt% based on weight of the solute composition; or
(iii) the antioxidant comprises BHT and the antioxidant is present in an amount ranging from 0.5 wt% to 10 wt% or from 1 wt% to 5 wt% based on weight of the solute composition.

## Patentansprüche

1. Spender, umfassend:
einen flexiblen Behälter mit einem geschlossenen Ende und einem gegenüberliegenden offenen Ende;
einer osmotisch wirksamen Lösungsproduktzusammensetzung, die wenigstens einen Abschnitt des Behälters umschließt, wobei die Lösungsproduktzusammensetzung umfasst:
- Antioxidans in einer Menge von 0,5 Gew.-% bis 20 Gew.-%, auf Grundlage des Gewichts der Lösungsproduktzusammensetzung, wobei das Antioxidans wenigstens ein Mitglied umfasst, das aus butyliertem Hydroxytoluol (BHT), butyliertem Hydroxyanisol (BHA), Thioethern, Histidin, Cystein, Tryptophan, Tyrosin, Ascorbinsäure, Vitamin E, Ascorbylpalmitat, Erythorbinsäure, Kaliummetabisulfit, Propylgallat, Natriumascorbat, Natriumbisulfat, Natriummetabisulfit, Natriumsulfit, Natriumformaldehydsulfoxylat und Thymol ausgewählt ist,
- ein Polymer, das wenigstens eines von Polyethylenoxid und Polyethylenglykol umfasst, und
- ein Salz;
eine semipermeable Membran, die die osmotisch wirksame Lösungsproduktzusammensetzung umschließt;
eine Kappe mit einer dichtenden Oberfläche, die zum dichtenden Eingreifen in das offene Ende des Behälters angepasst ist; und
eine Öffnung, die sich von einem Inneren des Behälters und durch die Kappe erstreckt.

2. Spender nach Anspruch 1, wobei das Antioxidans BHT umfasst.

3. Spender nach Anspruch 1 oder 2, wobei der flexible Behälter als ein Beutel ausgebildet ist, der angepasst ist, um eine Fluidzusammensetzung zu enthalten.

4. Spender nach Anspruch 3, wobei der Spender einen Durchflussregler umfasst, der ein starres zylindrisches Rohr und die Kappe umfasst, wobei das Rohr und die Kappe aus Kunststoff oder Metall hergestellt sind.

5. Spender nach einem der Ansprüche 1 bis 4, wobei die semipermeable Membran wenigstens ein Polymer umfasst, das ausgewählt ist, aus:
(i) Celluloseacetatbutyrat, Celluloseacetatpropionat, Polymethylmethacrylat und Ethylcellulose; oder
(ii) Celluloseacetatbutyrat und Mischungen aus Celluloseacetatbutyrat und Ethylcellulose; oder
(iii) Celluloseacetatpropionat und Mischungen aus Celluloseacetatpropionat und Ethylcellulose; oder
(iv) Polymethylmethacrylat und Mischungen aus Polymethylmethacrylat und Ethylcellulose.

6. Spender nach einem der Ansprüche 1 bis 5, wobei die semipermeable Membran ferner wenigstens ein Lösungsmittel umfasst, wobei das wenigstens ein Lösungsmittel ausgewählt ist, aus:
(i) Aceton, Methylenchlorid, Wasser und niederen Alkanolen mit 1-8 Kohlenstoffatomen; oder
(ii) Aceton, einer Mischung aus Aceton und Wasser, einer Mischung aus Aceton und Methanol und einer Mischung aus Aceton und Ethanol.

7. Spender nach einem der Ansprüche 1 bis 6, wobei der Behälter eine bogenförmige Außenkante aufweist, die an seinem offenen Ende neben seiner Verbindungsstelle mit der dichtenden Oberfläche der Kappe ausgebildet ist.

8. Spender nach Anspruch 7, wobei die gekrümmte Kante einen Krümmungsradius zwischen 0,254 mm (0,01 Zoll) und 2,29 mm (0,09 Zoll) oder zwischen 0,254 mm (0,01 Zoll) und 2,03 mm (0,08 Zoll) oder zwischen 0,245 mm (0,01 Zoll) und 1,78 mm (0,07 Zoll) aufweist.

9. Spender nach einem der Ansprüche 1 bis 8, der ferner einen Wirkstoff in dem flexiblen Behälter umfasst.

10. Spender nach einem der Ansprüche 1 bis 9 für die Verwendung bei der Abgabe des Wirkstoffs an ein Subjekt.

11. Spender nach einem der Ansprüche 1 bis 10, wobei das Salz NaCl umfasst.

12. Spender nach einem der Ansprüche 1 bis 11, wobei das Salz in einer Menge von 20 Gew.-% bis 60 Gew.-%, bezogen auf das Gewicht der Lösungsproduktzusammensetzung, vorhanden ist.

13. Spender nach einem der Ansprüche 1 bis 12, wobei das Polymer, das wenigstens eines von Polyethylenoxid und Polyethylenglykol umfasst, ein Polymer ist, das Polyethylenoxid umfasst, wobei das Polyethylenoxid optional in einer Menge im Bereich von 20 Gew.-% bis 55 Gew.-%, bezogen auf das Gewicht der Lösungsproduktzusammensetzung, vorhanden ist.

14. Spender nach einem der Ansprüche 1 bis 13, wobei das Polymer, das wenigstens eines von Polyethylenoxid und Polyethylenglykol umfasst, ein Polymer ist, das Polyethylenglykol umfasst, wobei das Polyethylenglykol optional in einer Menge im Bereich von 5 Gew.-% bis 25 Gew.-%, bezogen auf das Gewicht der Lösungsproduktzusammensetzung, vorhanden ist.

15. Spender nach einem der Ansprüche 1 bis 14, wobei
(i) das Antioxidans BHT umfasst; oder
(ii) das Antioxidans in einer Menge von 0,5 Gew.-% bis 10 Gew.-% oder von 1 Gew.-% bis 5 Gew.-%, bezogen auf das Gewicht der Lösungsproduktzusammensetzung, vorhanden ist; oder
(iii) das Antioxidans BHT umfasst und das Antioxidans in einer Menge von 0,5 Gew.-% bis 10 Gew.-% oder von 1 Gew.-% bis 5 Gew.-%, bezogen auf das Gewicht der Lösungsproduktzusammensetzung, vorhanden ist.

## Revendications

1. Organe d'administration comprenant :
un réservoir flexible ayant une extrémité fermée et une extrémité opposée ouverte ;
une composition de soluté osmotiquement efficace encapsulant au moins une partie du réservoir, la composition de soluté comprenant :
- un antioxydant dans une quantité allant de 0,5 % en poids à 20 % en poids, par rapport au poids de la composition de soluté, ledit antioxydant comprenant au moins un élément choisi parmi l'hydroxytoluène butylé (BHT), l'hydroxyanisole butylé (BHA), les thioéthers, l'histidine, la cystéine, le tryptophane, la tyrosine, l'acide ascorbique, la vitamine E, le palmitate d'ascorbyle, l'acide érythorbique, le métabisulfite de potassium, le gallate de propyle, l'ascorbate de sodium, le métabisulfite de potassium, le sulfite de sodium, le sulfoxylate de formaldéhyde sodique et le thymol,
- un polymère comprenant l'oxyde de polyéthylène et/ou le polyéthylène glycol, et
- un sel ;
une membrane semiperméable encapsulant la composition de soluté osmotiquement efficace ;
une capsule ayant une surface d'étanchéité apte à un contact étanche avec l'extrémité ouverte du réservoir ; et
un orifice s'étendant depuis l'intérieur du réservoir et traversant la capsule.

2. Organe d'administration selon la revendication 1, dans lequel l'antioxydant comprend du BHT.

3. Organe d'administration selon la revendication 1 ou 2, dans lequel le réservoir a la forme d'un sac apte à contenir une composition fluide.

4. Organe d'administration selon la revendication 3, ledit organe d'administration comprenant un modérateur de flux qui comprend un tube cylindrique rigide et la capsule, ledit tube et ladite capsule étant fabriqués à partir de plastiques ou de métaux.

5. Organe d'administration selon l'une quelconque des revendications 1 à 4, dans lequel la membrane semiperméable comprend au moins un polymère qui est choisi parmi :
(i) le butyrate d'acétate de cellulose, le propionate d'acétate de cellulose, le polyméthylméthacrylate et l'éthylcellulose, ou
(ii) le butyrate d'acétate de cellulose et les mélanges de butyrate d'acétate de cellulose et d'éthylcellulose, ou
(iii) le propionate d'acétate de cellulose et les mélanges de propionate d'acétate de cellulose et d'éthylcellulose, ou
(iv) le polyméthylméthacrylate et les mélanges de polyméthylméthacrylate et d'éthylcellulose.

6. Organe d'administration selon l'une quelconque des revendications 1 à 5, dans lequel la membrane semiperméable comprend en outre au moins un solvant, ledit au moins un solvant étant choisi parmi :
(i) l'acétone, le chlorure de méthylène, l'eau et un alcanol inférieur ayant de 1 à 8 atomes de carbone, ou
(ii) l'acétone, un mélange d'acétone et d'eau, un mélange d'acétone et de méthanol, et un mélange d'acétone et d'éthanol.

7. Organe d'administration selon l'une quelconque des revendications 1 à 6, dans lequel le réservoir a un bord extérieur arqué formé à son extrémité ouverte adjacente à sa jonction avec la surface d'étanchéité de la capsule.

8. Organe d'administration selon la revendication 7, dans lequel le bord arqué a un rayon de courbure d'entre 0,254 mm (0,01 pouce) et 2,29 mm (0,09 pouce) ou d'entre 0,254 mm (0,01 pouce) et 2,03 mm (0,08 pouce) ou d'entre 0,245 mm (0,01 pouce) et 1,78 mm (0,07 pouce).

9. Organe d'administration selon l'une quelconque des revendications 1 à 8, comprenant en outre un agent actif dans le réservoir flexible.

10. Organe d'administration selon l'une quelconque des revendications 1 à 9 pour utilisation dans l'administration d'un ingrédient actif à un sujet.

11. Organe d'administration selon l'une quelconque des revendications 1 à 10, dans lequel le sel comprend du NaCl.

12. Organe d'administration selon l'une quelconque des revendications 1 à 11, dans lequel le sel est présent dans une quantité allant de 20 % en poids à 60 % en poids par rapport au poids de la composition de soluté.

13. Organe d'administration selon l'une quelconque des revendications 1 à 12, dans lequel le polymère comprenant l'oxyde de polyéthylène et/ou le polyéthylène glycol est un polymère qui comprend l'oxyde de polyéthylène, ledit oxyde de polyéthylène étant facultativement présent dans une quantité allant de 20 % en poids à 55 % en poids par rapport au poids de la composition de soluté.

14. Organe d'administration selon l'une quelconque des revendications 1 à 13, dans lequel le polymère comprenant l'oxyde de polyéthylène et/ou le polyéthylène glycol est un polymère qui comprend le polyéthylène glycol, ledit polyéthylène glycol étant facultativement présent dans une quantité allant de 5 % en poids à 25 % en poids par rapport au poids de la composition de soluté.

15. Organe d'administration selon l'une quelconque des revendications 1 à 14, dans lequel
(i) l'antioxydant comprend le BHT, ou
(ii) l'antioxydant est présent dans une quantité allant de 0,5 % en poids à 10 % en poids ou de 1 % en poids à 5 % en poids par rapport au poids de la composition de soluté, ou
(iii) l'antioxydant comprend du BHT et l'antioxydant est présent dans une quantité allant de 0,5 % en poids à 10 % en poids ou de 1 % en poids à 5 % en poids par rapport au poids de la composition de soluté.
